(19) Europäisches Patentamt
European Patent Office
Office européen des brevets

(11) EP 2 895 221 B1

(12) EUROPEAN PATENT SPECIFICATION

(45) Date of publication and mention
of the grant of the patent:
**18.05.2016  Bulletin 2016/20**

(21) Application number: **13759994.0**

(22) Date of filing: **04.09.2013**

(51) Int Cl.:
*A61M 5/32* (2006.01)          *A61M 5/158* (2006.01)
*A61M 5/162* (2006.01)         *A61M 25/00* (2006.01)
*B82Y 30/00* (2011.01)         *A61B 5/15* (2006.01)

(86) International application number:
**PCT/EP2013/068227**

(87) International publication number:
**WO 2014/040886 (20.03.2014 Gazette 2014/12)**

(54) **MEDICAL INJECTION DEVICE**

MEDIZINISCHE INJEKTIONSVORRICHTUNG

DISPOSITIF D'INJECTION MÉDICAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.09.2012  EP 12184405
14.09.2012  US 201261700995 P**

(43) Date of publication of application:
**22.07.2015  Bulletin 2015/30**

(73) Proprietor: **Fresenius Kabi Deutschland GmbH
61352 Bad Homburg v.d.H. (DE)**

(72) Inventors:
• **BREUER-THAL, Barbara
65510 Idstein (DE)**
• **WITT, Ruediger
24229 Strande (DE)**
• **WEDER, Christoph
CH-3186 Düdingen (CH)**
• **FOSTER, Earl Johan
CH-1700 Fribourg (CH)**
• **JORFI, Mehdi
CH-1700 Fribourg (CH)**
• **ROBERTS, Matthew Neal
CH-1700 Fribourg (CH)**

(56) References cited:
**WO-A1-2004/037325     WO-A1-2006/074948
WO-A2-02/081013        US-A- 4 846 812
US-B2- 7 435 240**

**Description**

The invention relates to a medical injection device according to the preamble of claim 1, a method for producing a medical injection device and a method for inducing a stiffness change in a medical injection device.

A medical injection device of this kind having switchable mechanical properties comprises a hollow tube section having an inner bore, wherein the hollow tube section at least partially is made of a material exhibiting switchable mechanical properties such that the material in a first switching state comprises a first stiffness characterized by a first tensile storage modulus, in a second switching state comprises a second stiffness characterized by a second tensile storage modulus smaller than the first tensile storage modulus and is switchable between the first switching state and the second switching state by exposing the material to a stimulus.

Many current therapeutic and diagnostic medical practices rely on the sustained administration of intravenous fluids. This requires patients to keep a medical injection device such as a needle, cannula, catheter or other pipe or tube submerged in their body tissue for long periods of time, up to several days. Steel needles, whose use is a well-established standard practice, are known to inflame tissue surrounding the needle, causing discomfort for the patient, and they pose the risk of unwanted needlestick injury. In response to these drawbacks, soft or mechanically adaptive cannulas have been proposed with the objective to reduce tissue irritation and increase safety, but currently are not commercially available.

In order for a soft cannula to function effectively, there is a desire for a medical injection device having mechanical characteristics, in particular a sufficient rigidity while outside the body, to allow for insertion of the cannula.

From US 4,755,173 an intrinsically soft cannula is known which is composed of Teflon® or a hydrogel material and can be inserted into the body with the aid of a protruding steel needle tip that can be withdrawn after insertion of the cannula. This general approach nowadays has become standard of care.

Also, mechanically adaptive cannulas have become available which can transition from a rigid pre-insertion state to a flexible post-insertion state, thus eliminating the need for a steel needle tip. For example, US 7,435,240 suggests the use of shape-memory materials that require application of either electricity or radiation to induce a change in the elastic modulus, but no such materials are specified in the application. US 7,513,891 teaches the use of temperature-sensitive materials that require cooling at or below 0 °C to generate a rigid pre-insertion state and which soften upon insertion after warming to body temperature. WO 2006/074948 A1 claims that a material made of a block copolymer would be suitable to undergo a mechanical transition in response to contact with a fluid.

Currently, there appears to be a general understanding that an E-Modulus range of 3-5 GPa is ideal for the original (stiff) state of the adaptive needle and that for the soft state (i.e., after injection) a range of 500-700 MPa is desirable. There, however, may be situations in which a material with an initial stiffness characterized by a tensile storage modulus E' of ~5 GPa may not be suitable for a medical injection device to reliably allow penetration of human skin or tissue.

It is an object of the instant invention to provide a medical injection device, a method for producing a medical injection device and a method for inducing a stiffness change in a medical injection device which in an easy manner allow for a switching of a medical injection device between two switching states, the medical injection device exhibiting beneficial mechanical properties both in the first switching state for injecting the device and in the second switching state when resting for example in a human body.

This object is achieved by the subject-matter as defined by claims 1, 15 and 16. In addition, further advantageous embodiments follow from the dependent claims.

Accordingly, for producing the medical injection device a nanocomposite material is used comprising a matrix polymer and a nanoparticle network. The nanoparticle network is formed by a formation of a substantially three-dimensional network of nanoparticles which are incorporated, for example dispersed, in the matrix polymer and interact with each other and/or with the matrix polymer. The polymer nanocomposite in a first switching state comprises a first stiffness characterized by a first tensile storage modulus and in a second switching state comprises a smaller, second stiffness characterized by a second tensile storage modulus. The nanocomposite is switchable from the first switching state to the second switching state by exposing the polymer nanocomposite to a stimulus that influences interactions among the nanoparticles and/or between the nanoparticles and the matrix polymer.

A nanocomposite of this kind is for example known from US 2009/0318590 A1. US 2009/0318590 A1 however does not suggest the use of polymer nanocomposite materials for producing medical injection devices.

The instant invention is based on the idea to prepare medical injection devices, such as needles, cannulas, catheters or other pipes or tubes for injection into a patient, using polymer-based nanocomposites which are comprised of a polymeric matrix and reinforcing nanoparticles, whose interactions are stimuli-responsive and regulate the mechanical properties of the bulk material.

Although many potential nanofillers exist, in one embodiment crystalline cellulose nanoparticles, referred to as "nanowhiskers", may be used. Cellulose nanowhiskers (NWs) can be isolated from a variety of sources, including plants (e.g. wood, cotton, or wheat straw), marine animals (tunicates), as well as bacterial sources, such as algae, fungi, and amoeba (protozoa). Using NWs isolated from tunicates for example, materials may arise displaying enhanced mechanical properties, which can be explained with the formation of a percolating, hydrogen-bonded network of NWs within the polymer matrix. NW-based nanocomposites have the advantages of low cost, outstanding mechanical properties, availability, sustainability, biodegradability, and low density of NWs.

The stiffness of NW-based nanocomposites can be - preferably reversibly - changed by controlling the degree of interactions between the rigid filler (compare for example Capadona, J. R. et al., Science, 2008, 319, 1370. The NWs form a percolating network within the matrix which is - in the absence of a competitive hydrogen bonding agent - held together by hydrogen bonds among the surface hydroxyl groups. This causes a reinforcement of the polymer matrix. Upon exposure to chemicals that can competitively hydrogen-bond to the NWs, e.g. water, the interactions between individual NWs are reduced and the nanocomposite softens considerably, as predicted by mechanical models.

In one embodiment, mechanically-adaptive nanocomposites based on a rubbery ethylene oxide-epichlorohydrin copolymer (EO-EPI) and cellulose nanowhiskers isolated from tunicates (TNWs), as described for example in US 2009/0318590 A1, may be used for the medical injection device. The dry EO-EPI/TNW nanocomposites exhibit an increase of the storage modulus E' from 3.7 MPa (neat EO-EPI) to 800 MPa at a NW content of 19% v/v at 25 °C. These materials undergo a pronounced and reversible modulus reduction from 800 to 20 MPa upon exposure to water. Plasticization of the polymer matrix upon aqueous swelling has been shown to reinforce the effect, for example in nanocomposites based on poly(vinyl acetate) (PVAc) and TNWs or CNWs. The PVAc/TNW nanocomposites showed an increase of the E' from 1.8 GPa (neat PVAc) to 5.2 GPa for a nanocomposite containing 16.5% v/v TNWs. Due to the glassy nature of the PVAc matrix, these materials exhibit a much higher initial stiffness than the EO-EPI based systems, but soften greatly (5.2 GPa to 12 MPa) upon exposure to water due to matrix plasticization and TNW decoupling.

The realization of medical injection devices may possibly benefit if mechanically-adaptive materials with a stiffness characterized by a tensile storage modulus (E') of greater than 3.5 GPa, preferably 5 GPa, more preferably 6 GPa, most preferable 9 GPa in the first switching state are used. Such stiffness may be beneficial for injecting a device into for example a human skin allowing for an easy and safe penetration with a reduced risk of breakage of the device during the injection procedure.

Furthermore, the nanocomposite beneficially exhibits a tensile storage modulus of less than 1 GPa, preferably less than 500 MPa, more preferably less than 200 MPa, most preferably less than 100 MPa, in particular 20 MPa, in the second switching state. In this way the medical injection device exhibits in the second switching state of the nanocomposite material a significantly reduced stiffness compared to the first switching state such that the injection device is soft and flexible upon injection into a body and exposure to a suitable stimulus such as body fluid or tissue.

The stimulus serves to influence interactions among the nanoparticles and/or between the nanoparticles and the matrix polymer. By means of the stimulus, hence, the mechanical properties of the polymer nanocomposite are altered by influencing the interactions of the constituents of the polymer nanocomposites, i.e. the interactions between nanoparticles between themselves and between the nanoparticles and the matrix polymer. Due to the effect of the stimulus on the interactions between the nanoparticles among each other and/or with the matrix polymer the stiffness of the polymer nanocomposite is changed, allowing for a switching between the first switching state and the second switching state.

The switching herein is preferably reversible. Hence, the polymer nanocomposite may be switched from the first switching state to the second switching state and vice versa.

Although the switching preferably is reversible, it shall be noted that in principle this is not necessary. It also is conceivable that the injection device can be switched only in one direction from the first switching state to the second switching state, hence from rigid to soft.

The first tensile storage modulus in the first switching state of the polymer nanocomposite is preferably measured in a dry state at room temperature, i.e. 25°C. The second tensile storage modulus is preferably measured when the polymer nanocomposite is subjected to the stimulus, for example a body fluid, at the temperature of the stimulus, for example 37°C.

The polymer nanocomposite preferably is obtainable applying a process comprising the steps of:

- providing the matrix polymer,
- incorporating the nanoparticle network into the matrix polymer to obtain an intermediate mixture,
- subjecting the intermediate mixture to a heat treatment step involving exposure to a temperature equal to or greater than 100°C.

For example, in one embodiment, a matrix polymer may be provided dissolved in a fluid such as deionized water. Nanoparticles may in one embodiment be provided for example in the shape of powder in a lyophilized or spray-dried state and may be dispersed in a fluid such as deionized water. Both the solution of the matrix polymer and the dispersion of the nanoparticles may be obtained by techniques known to the person skilled in the art, for example by a stirring and/or sonicating for a predetermined amount of time at a predetermined temperature possibly greater than room temperature. Also, the mixture of the matrix polymer and the nanoparticles may be obtained by a suitable mixing technique known to the person skilled in the art, such as stirring or sonication or both, resulting in a homogeneous mixture.

The heat treatment step may for example include a compression molding of the polymer nanocomposite at a temperature equal to or greater than 100°C. The compression molding may for example take place at a temperature between 100°C and 160°C, for example at 120°C or 150°C, preferably at a temperature below the melting temperature of the polymer nanocomposite. During compression molding a pressure, for example between 500 psi and 5000 psi, for example 1000 psi or 2000 psi, is applied to the intermediate mixture of the matrix polymer and the nanoparticles for a predetermined amount of time. For example, in a first step a pressure of 1000 psi may be applied for a duration of 1 to 20 minutes, for example 2 minutes, followed by a second step in which a pressure of 2000 psi for a duration of 5 to 45 minutes, for example 15 minutes, is applied, hence applying different pressures for different amounts of time.

After the heat treatment step the resulting polymer nanocomposite may be allowed to rest for a predetermined amount of time, for example 30 to 150 minutes, for example 90 minutes, possibly at an elevated temperature smaller than the temperature applied during the heat treatment step, for example 70 °C.

Prior to the heat treatment step, the intermediate mixture obtained by incorporating the nanoparticles into the matrix polymer is preferably subjected to a drying step involving exposure to an elevated temperature of 30 °C to 80 °C over an elongated period of time of for example one day to 10 days. For example, the drying step may include a first drying step including exposure to a first drying temperature of 30 °C to 50 °C, for example 35 °C, over a first period of time of one day to 10 days, for example five days. The drying step may further include a second drying step including exposure to a second drying temperature of 50 °C to 80 °C, for example 70 °C, over a second period of time of five hours to 48 hours, for example 24 hours. Such drying steps serve to evaporate water from the mixture of the matrix polymer and the nanoparticles and may be carried out in a suitable oven into which the mixture of the matrix polymer and the nanoparticles is placed.

The exposure of the mixture of the matrix polymer and the nanoparticles to an elevated temperature of equal to or greater than 100°C during the heat treatment step has a twofold effect. In this regard it was surprisingly found that a heat treatment of the mixture of the matrix polymer and the nanoparticles dispersed therein may serve to increase the stiffness of the resulting polymer nanocomposite in the first switching state, hence yielding a material which exhibits a large stiffness in its first, initial switching state. Furthermore, the heat treatment step may also serve to adjust the stiffness in the soft, second switching state, as well as the swelling characteristics of the polymer nanocomposite when subjected to a stimulus in the shape of a water-containing fluid.

The matrix polymer, in a preferred embodiment, comprises a - preferably highly - polar polymer capable of forming non-covalent interactions, in particular hydrogen bonds, with the nanoparticles. For example, the matrix polymer may comprise vinyl polymers such as polyvinyl alcohol (PVOH), poly(acrylic acid), poly(acryl amide)s, poly(vinyl pyridine), copolymers of these respective monomers and other monomers, polycondensates such as polyamides and polyesters, polysaccharides such as cellulose, starch, alginates, pectins, hyaluronane, chitin, chitosan and their derivatives, proteins, and other polar polymers. By forming non-covalent interactions such as hydrogen bonds with the nanoparticles, the matrix polymer in the first switching state may interact with the nanoparticles yielding an increased stiffness in the first switching state. Such bonds are effected by a stimulus and are at least partly released when subjecting the polymer nanocomposite to the stimulus such that the stiffness of the polymer nanocomposite is decreased yielding a significantly reduced stiffness in the second switching state.

The nanoparticles are advantageously capable of forming non-covalent interactions, such as hydrogen bonds, with each other and/or with the matrix polymer.

In an advantageous embodiment, nanofibers having a length which is large with respect to their width are used as nanoparticles. In one embodiment, cellulose nanowhiskers such as nanowhiskers derived from tunicates (tunicate nanowhiskers, TNW) or from cotton (cotton nanowhiskers, CNW) may be used as nanofibers.

TNWs are advantageously isolated from tunicates by preparing a cellulose pulp and by applying a hydrolysis, for example a sulphuric acid hydrolysis, to the cellulose pulp.

CNWs may for example be obtained by isolation employing for example a hydrolysis, for example a sulphuric acid hydrolysis, and dialysis treatment, followed for example by a sonication for a predetermined amount of time, for example three hours, and a settlement period of for example 18 hours. The resulting supernatant may then be decanted off, and the resulting CNW dispersion may be dried, for example spray-dried to yield a dried CNW powder.

Advantageously, nanofibers comprising an aspect ratio (ratio between length and diameter) greater than 5, preferably greater than 10, more preferably greater than 50, most preferably greater than 80 are employed. For example, CNWs exhibit an aspect ratio of for example about 10 (having a length of about $200 \pm 70$ nm and a diameter (width) of about $22 \pm 6$ nm). TNWs exhibit an aspect ratio of for example about 83 (having a length of for example about $2500 \pm 1000$ nm and a diameter (width) of about $30 \pm 5$ nm).

The polymer nanocomposite, as substantial constituents, comprises a matrix polymer, for example PVOH, and nanoparticles. In this regard, the polymer nanocomposite may comprise in one embodiment a content of 1 % to 30 %, preferably 2 % to 25 %, more preferably 3 % to 20 % v/v of nanoparticles (v/v indicates the volume fraction defined as the volume of a constituent divided by the volume of all constituents of a mixture prior to mixing, in contrast to w/w which indicates the weight fraction). Dependent on the exact content fraction of the nanoparticles in the matrix polymer the mechanical properties of the polymer nanocomposite in the first switching state and in the second switching state may fine-tuned. In general, a larger fraction of nanoparticles in the polymer nanocomposite yields a larger stiffness both in the first switching state and in the second switching state.

The polymer nanocomposite may - besides the matrix polymer and the nanoparticles - comprise further constituents such as - but not limited to - plasticizers, process agents, stabilizers and/or other filling material.

As mentioned already above, the switching between the switching states is preferably reversible. Hence, for switching the polymer nanocomposite from the first switching state to the second switching state a stimulus reduces the interactions among the nanoparticles and/or between the nanoparticles and the matrix polymer, thus reducing the stiffness of the polymer nanocomposite. Vice versa, for switching the polymer nanocomposite from the second switching state to the first switching state a stimulus enhances the interactions among the nanoparticles and/or between the nanoparticles and the matrix polymer, thus increasing the stiffness of the polymer nanocomposite.

Herein, the stimulus for switching the polymer nanocomposite from the first switching state to the second switching state may, in one embodiment, include subjecting the polymer nanocomposite to a water-containing composition, in particular a water-containing fluid, for example a body fluid. The water-containing composition herein may have an elevated temperature of for example 30 °C to 50 °C, preferably 35 °C to 40 °C, for-example 37 °C.

The switching between the first switching state and the second switching state takes place over time and hence must be considered as a process. It can be imagined that the switching processed may be stopped after some time prior to reaching a final switching state, the polymer nanocomposite then adopting an intermediate switching state in between the first switching state and the second switching state. In this regard, the first switching state and the second switching state represent extreme states prior or after switching which the polymer nanocomposite may adopt.

The process may be reversed by simply removing the stimulus which is applied for switching form the first to the second switching state. For example, the polymer nanocomposite may be switched from the second switching state to the first switching state by drying the water-containing nanocomposite, either at ambient conditions, or under vacuum and/or at an elevated temperature, or under other suitable conditions

The ratio of the storage moduli of the two switching states may, in one embodiment, be greater than 20, preferably greater than 50, more preferably greater than 100, and most preferably greater then 500. For example, a polymer nanocomposite using PVOH as matrix polymer and nanoparticles in the shape of tunicate nanowhiskers (TNWs) or cotton nanowhiskers (CNWs) may exhibit a change of storage moduli from 6,8-13,7 GPa in the first switching state to 2-160 MPa in the second switching state.

The nanoparticle network formed by the formation of nanoparticles is substantially three-dimensional, hence yielding essentially isotropic mechanical properties of the polymer nanocomposite. In particular, the stiffness characterized by the tensile storage modulus in the first switching state and in the second switching state is substantially equal in all three spatial directions, such that no preferred direction with an increased tensile storage modulus exists.

Those skilled in the art will appreciate that other embodiments may rely on the same principle. For example, a nanocomposite may be produced from a matrix polymer that has suitable groups for pi-pi-interactions and nanoparticles which are suitably functionalized so that they can exhibit pi-pi interactions with the polymer and/or other nanoparticles. Exposure to an agent that can form pi-pi interactions with the polymer and/or the nanoparticles can then serve to switch between said two switching states.

Those skilled in the art will also know that other embodiments may rely on appropriately functionalized nanoparticles other than cellulose nanowhiskers, for example, but not limited to, boemite whiskers, carbon nanotubes, and nanoparticles made from synthetic or natural polymers.

In one embodiment, the hollow tube section of the medical injection device has a wall thickness between 50 $\mu$m and 1000 $\mu$m, preferably between 100 $\mu$m to 500 $\mu$m. The wall thickness herein has an influence for example on the overall strength of the medical injection device, which in particular is relevant when injecting the device into a patient. Furthermore, the wall thickness also has an influence on the switching speed of the medical injection device. In this regard it generally can be said that - due to the diffusion speed within the nanocomposite - the switching speed increases with decreasing wall thickness such that a device having a thin wall thickness may switch in a shorter time from the first switching state to the second switching state when subjected to an appropriate stimulus than a device having a larger wall thickness. Vice versa the switching speed decreases with increasing wall thickness. Accordingly, the wall thickness may for example be chosen to achieve a sufficient strength for an envisioned application while at the same time obtaining a suitable switching speed of for example several minutes.

The object is also achieved by a method for producing a medical injection device having switchable mechanical properties, comprising a hollow tube section having an inner bore, wherein the hollow tube section at least partially is made of a material exhibiting switchable mechanical properties such that the material in a first switching state comprises a first stiffness characterized by a first tensile storage modulus, in a second switching state comprises a second stiffness characterized by a second tensile storage modulus smaller than the first tensile storage modulus and is switchable from the first switching state to the second switching state by exposing the material to a stimulus. Herein, said material comprises a polymer nanocomposite which is produced by providing a matrix polymer and incorporating a nanoparticle network into the matrix polymer, wherein the nanoparticle network is formed by a formation of a substantially three-dimensional network of nanoparticles which are incorporated, for example dispersed, in the matrix polymer and interact with each other and/or with the matrix polymer, wherein said stimulus influences interactions among the nanoparticles and/or between the nanoparticles and the matrix polymer.

The object is furthermore achieved by a method for inducing a stiffness change in a medical injection device having switchable mechanical properties and comprising a hollow tube section having an inner bore, wherein the hollow tube section at least partially is made of a material exhibiting switchable mechanical properties such that the material in a first switching state comprises a first stiffness characterized by a first tensile storage modulus, in a second switching state comprises a second stiffness characterized by a second tensile storage modulus smaller than the first tensile storage modulus and is switched from the first switching state to the second switching state by exposing the material to a stimulus. Herein, said material comprises a polymer nanocomposite comprising a matrix polymer and a nanoparticle network, wherein the nanoparticle network is formed by a formation of a substantially three-dimensional network of nanoparticles which are incorporated, for example dispersed, in the matrix polymer and interact with each other and/or with the matrix polymer, wherein said stimulus switches the polymer nanocomposite between the first switching state and the second switching state by influencing interactions among the nanoparticles and/or between the nanoparticles and the matrix polymer.

The advantages and advantageous embodiments described above for the polymer nanocomposite are applicable to such methods in an analogous fashion such that it shall be referred to the above explanations.

The idea underlying the invention shall subsequently be described in more detail with regard to the embodiments shown in the figures. Herein,

Fig. 1A, B     show AFM height images for lyophilized TNWs (Fig. 1A) and spray-dried CNWs (Fig. 1B) deposited from aqueous dispersions (0.1 mg/mL) onto freshly cleaved mica surfaces;

Fig. 2A-D     show dynamic mechanical analysis (DMA) data of dry PVOH and dry PVOH/NW nanocomposites as a function of temperature and NW content: Tensile storage moduli E' (Fig. 2A) and loss tangent tan $\delta$ (Fig. 2B) of PVOH/TNW nanocomposites; tensile storage moduli E' (Fig. 2C) and loss tangent tan $\delta$ (Fig. 2D) of PVOH/CNW nanocomposites;

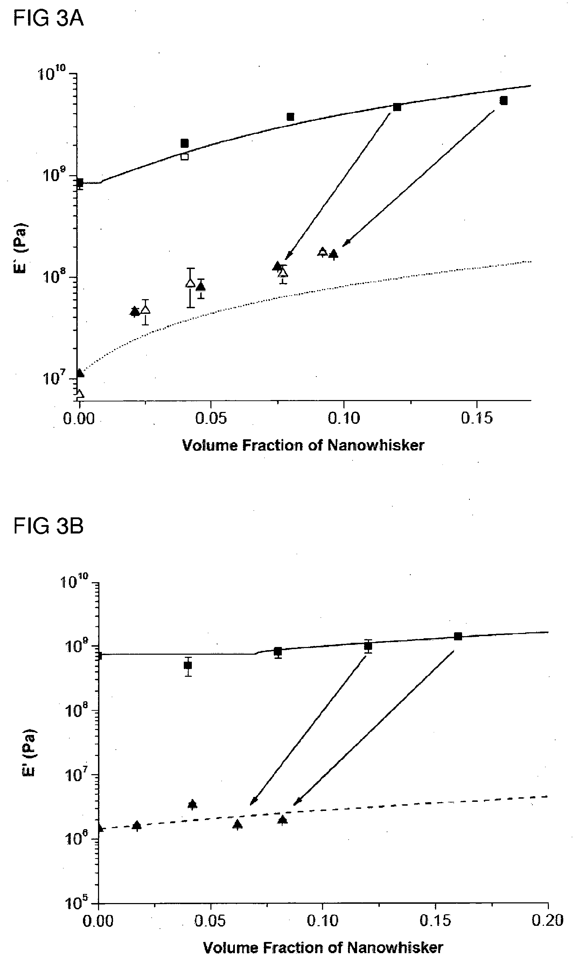

Fig. 3A     shows tensile storage moduli (E') of neat PVOH and PVOH/TNW nanocomposites as a function of NW content in the dry state at 100 °C (■), re-dried after swelling with ACSF for 1 week (□), ACSF-swollen after immersion in ACSF at 37 °C for 1 week (▲) and 1 month (△) (the solid line shows values predicted by the percolation model for the dry state ($E'_r$= 80 GPa), the dotted line shows values predicted by the Halpin-Kardos model for samples conditioned in ACSF at 37 °C ($E'_{lr}$ = 130 GPa, $E'_{tr}$ = 5 GPa, $E'_s$ = 11 MPa, $G'_r$ = 1.77 GPa, $G's$ = 3.9 MPa, $v_r$ = 0.3, $v_s$ = 0.44));

Fig. 3B     shows tensile storage moduli (E') of neat PVOH and PVOH/CNW nanocomposites as a function of NW content in the dry state at 100 °C (■), and ACSF-swollen after immersion in ACSF at 37 °C for 1 week (▲) (the solid line shows values predicted by the percolation model ($E'_r$ = 10 GPa), the dotted line shows the prediction by the Halpin-Kardos model for samples conditioned in ACSF at 37 °C ($E'_{lr}$ = 130 GPa, $E'_{tr}$ = 5 GPa, $E'_s$ = 1.44 MPa, $G'_r$ = 1.77 GPa, $G'_s$ = 0.5 MPa, $v_r$ = 0.3, $v_s$ = 0.44); because of solvent uptake the volume fraction of NWs in ACSF-swollen samples is lower than in the dry state; Data points represent averages of N = 3-6 measurements $\pm$ s.d.);

Fig. 4     shows data indicating the swelling of PVOH/TNW nanocomposites compression-molded at 150 °C (■), PVOH/CNW nanocomposites compression-molded at 120 °C (●), a PVOH/TNW nanocomposite compression-molded at 120 °C (○), and a PVOH/CNW nanocomposite compression-molded at 150 °C (□) as a function of NW content, after the samples were immersed in ACSF at 37 °C for 1 day (data represent averages of N = 3 measurements $\pm$ s.d.);

Fig. 5A, B     show dynamic mechanical analysis (DMA) data of ACSF-swollen films of neat PVOH and PVOH/TNW nanocomposites (Fig. 5A) and PVOH/CNW nanocomposites (Fig. 5B) as a function of temperature and NW content after immersion in ACSF at 37 °C for 1 week;

Fig. 6A     shows a schematic drawing illustrating the interactions between PVOH and NWs;

Fig. 6B     shows an enlarged view of the drawing of Fig. 6A in the region A;

Fig. 7A-C    show conductometric titration curves of NWs: Lyophilized TNWs (Fig. 7A), spray-dried CNWs (Fig. 7B), and lyophilized CNWs (Fig. 7C);

Fig.8A-D    show representative scanning electron microscopy images of NWs (SEM, Fig. 8A) and transition electron microscopy images of NWs (TEM, Fig. 8B-D), wherein Fig. 8A and 8B show lyophilized TNWs, Fig. 8C shows spray-dried CNWs and Fig. 8D shows lyophilized CNWs, all deposited from aqueous dispersions (0.1 mg/mL);

Fig. 9A-B    show DSC thermograms (second heating) of PVOH/TNW nanocomposites (Fig. 9A) and PVOH/CNW nanocomposites (Fig. 9B), together with thermograms of neat PVOH films compression-molded at 150 °C (Fig. 9A) and 120 °C (Fig. 9B);

Fig. 10    shows DMA data of ACSF-swollen films of neat PVOH and PVOH/TNW nanocomposites after immersion in ACSF at 37 °C for 1 month;

Fig. 11    shows DMA data of ACSF-swollen films of 16% v/v PVOH/TNW nanocomposites after immersion in ACSF at 37 °C for 1 day (the processing temperature is indicated in the figure);

Fig. 12    shows DMA data of ACSF-swollen films of 16% v/v PVOH/CNW nanocomposites after immersion in ACSF at 37 °C for 1 day (the processing temperature is indicated in the figure);

Fig. 13    a schematic drawing of a medical injection device;

Fig. 14    a schematic drawing illustrating the process of injecting a medical injection device into the skin of a patient;

Fig. 15    tensile storage modulus E' of a PVOH film processed at 120 °C, as measured by DMA (the measurement was conducted at room temperature on a dry film, until at 46 minutes water was added and the sample was measured while submerged);

Table 1    states thermal properties of neat PVOH and PVOH/NW nanocomposites as a function of NW type and content;

Table 2    states tensile storage moduli (E') of dry and ACSF-swollen films of neat PVOH and PVOH/NW nanocomposites determined by DMA (data represent averages (N = 4-7));

Table 3    gives a comparison of tensile storage moduli (E') of current materials with previous mechanically-adaptive nanocomposites comprising ~16% v/v of NWs; and

Table 4    states swelling data of neat PVOH and PVOH/TNW nanocomposites at 37 °C in ACSF as a function of TNW content (data represent averages (N = 3) ± s.d.).

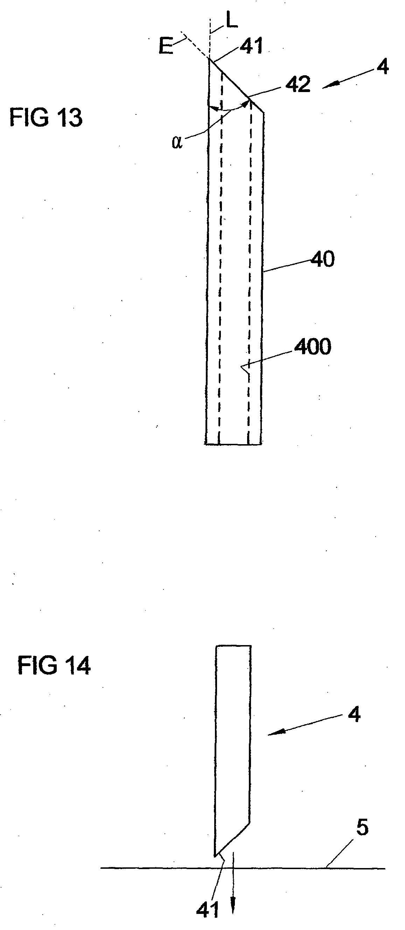

Fig. 13 shows in a schematic drawing a medical injection device in the shape of a needle 4 comprising a hollow tube section 40 having a longitudinal inner wall 400. The medical injection device 4, as commonly known, serves to be injected for example into a vein or other vessel of a patient by penetration of the skin and tissue of the patient. In the injected state the medical injection device 4 rests in the patient's body and may serve to inject a medical fluid into the patient or to withdraw a fluid, for example blood or another body fluid, from the patient.

The medical injection device comprises a sharpened tip 41, which - as schematically shown in Fig. 14 - serves to penetrate the patient's skin 5. At the tip 41, an opening 42 for fluid output or intake is arranged.

At the tip 41 the medical injection device 4 has an angle $\alpha$ measured between a cutting plane E and the direction of longitudinal extension L of the hollow tube section 40.

The medical injection device 4 may for example be part of an infusion set, a syringe, a catheter or any other medical device employing a cannula for access to a patient's body.

According to the instant invention, the medical injection device is at least partially made of a polymer nanocomposite material comprising a matrix polymer and a nanoparticle network. Herein, the nanoparticle network is formed by a formation of a substantially three-dimensional network of nanoparticles which at least partly are dispersed in the matrix polymer and interact with each other and/or with the matrix polymer. The polymer nanocomposite comprises two switching states. In a first switching state the polymer nanocomposite comprises a first stiffness characterized by a first tensile storage modulus. In a second switching state the polymer nanocomposite comprises a second stiffness characterized by a second tensile storage modulus which is smaller than the first tensile storage modulus. The polymer nanocomposite is switchable between the first switching state and the second switching state by exposing the polymer nanocomposite to a stimulus such as a water-containing composition, for example a body fluid or body tissue.

For a first exemplary embodiment of a medical injection device, compression-molded films of poly(vinyl acetate) (PVAc) with 16% v/v CNWs were prepared according to the method described by Shanmuganathan K. et al., "Bio-inspired mechanically-adaptive nanocomposites derived from cotton cellulose whiskers", J. Mater. Chem., 2010, 20, 180-186. The storage modulus (E') for dry samples of this mechanically adaptive nanocomposite is 4.0 GPa and the material softens upon exposure to emulated physiological conditions (exposure to artificial cerebrospinal fluid at 37 °C for 1 week) to exhibit an E' of 5 MPa. Medical injection devices were machined from the films and had the following dimensions: a thickness of 510 $\mu$m, a width of 3 mm, a length of 25 mm, and a sharpened tip 41 at one end cut at a angle $\alpha$ of 31° (compare Fig. 13).

"Woody nitrile extra" gloves were used as skin mimic according to the method by Kim, H.; Colton, J.S., Journal of Medical Engineering and Technology, 2005, 29, 181. The material was stretched to 166% of its normal size in a film stretcher. To test the effectiveness of the devices made, the skin mimic was manually punctured with the device, using approximately the same force and speed.

For a second embodiment, a compression-molded film of PVOH with 16% v/v CNWs, prepared by compression molding at 120 °C according to the protocol described in detail below, was used. The film had a thickness of 475 $\mu$m. The storage modulus (E') of dry samples of this mechanically adaptive nanocomposite is 9.0 GPa, and it softens upon exposure to emulated physiological conditions (exposure to artificial cerebrospinal fluid at 37 °C for 1 day) to exhibit an E' of 2 MPa.

For a third embodiment, a compression-molded film of PVOH with 1.5% v/v TNWs, prepared by compression molding at 150 °C according to the protocol described in detail below, was used. The film had a thickness of 300 $\mu$m. The storage modulus (E') of dry samples of this mechanically adaptive nanocomposite is 7.3 GPa.

For comparison, a film of commercially available PVOH, prepared by compression molding at 120 °C according to the protocol described in detail below, was used. The film had a thickness of 550 $\mu$m. The storage modulus (E') of dry samples of this known material is 7.0 GPa and it softens upon exposure to emulated physiological conditions (exposure to artificial cerebrospinal fluid at 37 °C for 1 day) to exhibit an E' of 1 MPa.

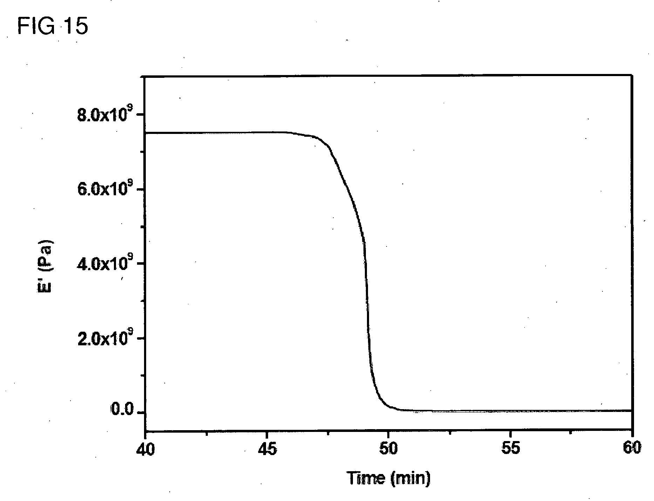

It is desirable for the rate of mechanical switching of the material to be rapid, preferably less than 10 minutes. The rate of mechanical switching of compression-molded films of neat PVOH was therefore determined by placing a dry film in the DMA submersion clamp without filling the submersion vessel with water. The sample had been prepared by compression molding at 120 °C according to the protocol described in detail below, and the film had a thickness of 80 $\mu$m. The tensile measurement was started, under isothermal conditions, to establish the storage modulus of the dry film. As shown in Fig. 15, for the sample under test the equilibriated value of the storage modulus after 45 minutes at room temperature was 7.5 GPa. At t = 46 minutes, preheated water (37 °C) was carefully added to the submersion vessel as rapidly as possible to a level that completely submerged the PVOH film. The tensile measurement was continued until an equilibrium state was reached, indicated by no further change in the storage modulus of the wet PVOH film. Within 8 minutes after exposure to water, the storage modulus of the film had decreased to approximately 1 MPa.

Nanocomposite materials suitable for use in medical injection devices shall subsequently be described in terms of mechanically-adaptive nanocomposites comprising poly(vinyl alcohol) (PVOH) and cellulose nanowhiskers (NWs), whose mechanical properties change significantly upon exposure to physiological conditions. It shall be noted, however, that the invention is not limited to the use of such particular composites described herein, but may also be implemented using other matrix polymer materials and/or nanoparticles.

PVOH/NW nanocomposites described in the following are produced using NWs derived from tunicates (TNWs) and cotton (CNWs) having different aspect ratios (TNWs = 83 and CNWs = 10), surface charge densities (TNWs = 90 mmol/kg and CNWs = 40 mmol/kg), and filler contents (0-16% v/v). Dynamic mechanical analysis results are presented in the following which show an enhancement of the tensile storage moduli (E') of the dry nanocomposites in comparison to the neat PVOH.

For example, as will be described in detail below, below the glass transition temperature (Tg ~70 °C) the tensile storage modulus E' of the dry PVOH/TNW nanocomposites increases from 7.3 GPa for the neat polymer to 13.7 GPa for the nanocomposite with 16% v/v TNWs. The stiffness of the materials is greatly reduced upon exposure to aqueous conditions, on account of reduced NW-NW and NW-matrix interactions. Immersing the nanocomposites into artificial cerebrospinal fluid (ACSF) at body temperature (37 °C) causes a drastic drop in E', for example from 13.7 GPa to 160 MPa in the case of the nanocomposites containing 16% v/v TNWs. Nanocomposites comprising 16% v/v CNWs exhibit an E' of 9.0 GPa in the dry state, which dropped to ~1 MPa upon exposure to artificial cerebrospinal fluid (ACSF) used for simulating physiological conditions. As will be described, the swelling characteristics of the materials, and therewith the extent of mechanical switching, is also influenced via the processing conditions.

The isolation, modification, characterization and application of cellulose nanofibers from plants (including wood, coconut husks, sisal, tunicates, cotton, ramie, straw, sugar beet, and many others) and animals (e.g. tunicates) is currently attracting significant attention, driven by the abundance and renewable nature of the biological sources and the attractive mechanical properties of nanocellulose. The latter originate from the hierarchical, uniaxially oriented structure of native cellulosic materials. Extended and aligned cellulose macromolecules (D-glucose units, which are condensed through $\beta(1\rightarrow4)$ glycosidic bonds) assemble into microfibrils, in which they are stabilized through hydrogen bonds. The microfibrils are largely crystalline, but they also contain regions that are less well ordered (i.e., largely amorphous). The cross-sectional dimension of the microfibrils ranges from 2-20 nm, depending on the origin of the cellulose. The elementary fibrils aggregate to form microfibrils with diameters of ~15-20 nm, which further aggregate into larger bundles and finally, with "blinders" consisting of amorphous lignin and hemicelluloses, into cellulosic fibers. These hierarchical structures can be deconstructed by mechanical and chemical processes, which yield highly crystalline cellulose nanofibers, collectively referred to as nanocelluloses or cellulose nanofibers.

Two main types of nanocelluloses can be distinguished - microfibrillated cellulose (MFC) and cellulose nanocrystals (CNC) - although it should be noted that differences in the isolation processes and the nature of the source also cause some variation within these families.

The isolation of MFC (also referred to as cellulose microfibrils, nanofibrillated cellulose, and cellulose nanofibrils) involves milling the native cellulose, followed by subsequent alkali and bleaching treatments to eliminate lignin and hemicelluloses. Mechanical disintegration of bleached wood pulp, which has traditionally been carried out in highpressure homogenizers, separates the microfibrils and affords MFC in the form of fibrils with a diameter of ~20 nm and a length of several micrometers. The "amorphous defects" present in the native microfibrils are retained and the fibrous nature gives rise to entangled, network-like structures.

CNCs (also referred to as cellulose whiskers or nanowhiskers (NWs), nanocrystalline cellulose (NCC), cellulose microcrystals, and rod-like cellulose crystals) are produced by the hydrolysis of the bleached cellulose pulp with mineral acids. The hydrolysis serves to remove the amorphous cellulose domains, thereby also reducing the molecular weight. The remaining particles are usually separated by ultrasonic treatment. The dimensions of the resulting rod-like CNC particles depend primarily on the structure in the native material (i.e. the source) and to a lesser extent also on the hydrolysis conditions employed. The diameter of CNCs is in the range of 5 - 25 nm and their length varies between 100 - 300 nm (in the case of wood and cotton) and several $\mu$m (in the case of tunicin). Since CNCs lack the amorphous domains present in the original microfibrils (and also in MFC), they display extremely high elastic moduli (100-150 GPa) and a high tensile strength (~10 GPa) in their long direction. A discussion of nanocellulose production and properties has been thoroughly treated in several reviews, such as Siqueira, G. et al., Polymers 2010, 2, 728 and Klemm, D. et al., Angew. Chem. Int. Ed. 2011, 50, 5438.

For the embodiments described below, nanowhiskers derived from tunicates (TNWs) or cotton (CNWs) have been employed, both representing types of cellulose nanocrystals (CNCs), although in principle also other types of nanofibers may be used.

Subsequently, in a first section experiments with regard to suitable nanocomposite materials for medical injection devices shall be described. In a second section, then, the results of the experiments shall be discussed.

EXPERIMENTS RELATING TO EMBODIMENTS OF NANOCOMPOSITES

Materials. Poly(vinyl alcohol) (PVOH) 99% hydrolyzed (Mw = 85,000-124,000 g/mol; $\nabla$ = 1.26 g/mL) and all reagents were purchased from Sigma-Aldrich and used without further purification. Artificial cerebrospinal fluid (ACSF) was prepared by dissolving the following materials in 1 L of deionized water: NaCl = 7.25 g, KCl = 0.22 g, NaHCO3 = 2.18 g, CaCl2·2H2O = 0.29 g, KH2PO4 = 0.17 g, MgSO4·7H2O = 0.25 g, and D-glucose = 1.80 g. A literature value of 1.46 g/mL was used for the density of the NWs.

**Isolation of Cellulose Nanowhiskers from Tunicates.** TNWs were isolated from tunicates (Styela clava) collected from floating docks in Point View Marina (Narragansett, RI). The TNWs were prepared by sulfuric acid hydrolysis of the cellulose pulp. The protocol was based on the method described in Favier et al., Macromolecules 1995, 28, 6365, utilized modifications as described in Shanmuganathan et al., ACS Appl. Mater.Interfaces 2010, 2, 165.

The bleached tunicate mantles were blended at high speed, yielding a fine cellulose pulp. Sulfuric acid (95-97%, 600 mL) was slowly (over the course of 2 h) added under vigorous mechanical stirring to an ice-cooled suspension of tunicate cellulose pulp in deionized water (6 g in 600 mL, 20 °C). After 500 mL of the acid had been added, the dispersion was removed from the ice bath and was heated to 40 °C during the addition of the final 100 mL of acid. After the acid addition was complete, the dispersion was heated to 60 °C and was kept at this temperature for 1 h under continuous stirring. The mixture was then cooled to room temperature, centrifuged (30 min at 3300 rpm), and the supernatant solution was decanted. Deionized water was added and the centrifugation step was repeated until the pH of the dispersion reached about 5. After the last centrifugation the resulting NWs were dialyzed in three successive 24 h treatments against deionized water to remove the last residues of the sulfuric acid. The suspension was diluted with deionized water (total volume 1 L) and sonicated for 18 h, before it was filtered through a No. 1 glass filter in order to remove any remaining aggregates. The concentration of the NWs in the final dispersion was determined gravimetrically to be ~3 mg/mL. This dispersion was freeze-dried using a VirTis BenchTop 2K XL lyophilizer with an initial temperature of 25 °C and a condenser temperature of -78 °C. The TNWs aerogel those produced was stored and used as needed.

**Isolation of Cellulose Nanowhiskers from Cotton.** CNWs were isolated from Whatman filter paper with minor modifications to a procedure published in Capadona, J. R. et al., Biomacromolecules, 2009, 10, 712. After sulfuric acid hydrolysis and dialysis treatment, the resulting dispersion was sonicated for 3 h and left to settle at room temperature for 18 h. The supernatant was then decanted off and the CNW dispersion was spray-dried using a Büchi Mini Spray Dryer (Model B-191) to yield dried CNWs as a white powder. The drying parameters were an inlet temperature of 110 °C, a flow rate of 4 mL/min, a nozzle airflow of 700 mL/min, an aspiration rate of 70%, and an outlet temperature of 60 °C.

**Preparation of PVOH/NW Nanocomposites.** Lyophilized TNWs or spray-dried CNWs were dispersed in deionized water at a concentration of 5 mg/mL by sonicating for 10 h and 7 h, respectively. PVOH was dissolved in deionized water at a concentration of 50 mg/mL by stirring for 2 h at 90 °C. Nanocomposites comprising 4-16% v/v NWs were prepared by combining the appropriate amounts of the NW dispersion and PVOH solution to cast a film weighing 1 g. This mixture was stirred at room temperature for 30 min, followed by sonication for 30 min, and the resulting homogeneous mixtures were cast into Teflon Petri dishes of a diameter of 100 mm. The dishes were placed into an oven at 35 °C for 5 days to evaporate the water, and the resulting films were then further dried in the oven at 70 °C for 24 h. The films were compression-molded between spacers in a Carver laboratory press (1000 psi for 2 min, followed by an increase of pressure to 2000 psi for 15 min). Unless otherwise stated, PVOH/TNW films were compression-molded at 150 °C, and PVOH/CNW films were compression molded at 120 °C. Both types of films were allowed to cool to ~70 °C over ca. 90 min under the applied pressure to yield 70-110 $\mu$m thin nanocomposite films. For reference purposes, neat PVOH films were prepared in a similar manner by solution-casting and subsequent compression-molding at 120 °C and 150 °C.

**Scanning Electron Microscopy (SEM).** The morphology of the NWs was examined by scanning electron microscopy (SEM) using a FEI XL 30 SIRON FEG microscope. A droplet of dilute aqueous NW dispersions (0.1 mg/mL) were deposited on a silicon wafer (TEDPELLA, Inc.) and allowed to dry. Then the samples were coated with a layer of gold (~5 nm), and observed with an accelerating voltage of 5 kV.

**Transmission Electron Microscopy (TEM).** For TEM studies, 3 $\mu$L of dilute aqueous NW dispersions (0.1 mg/mL) were deposited on carbon-coated grids (Electron Microscopy Sciences) and allowed to dry. The samples were examined with a Philips CM100 Biomicroscope operated at an accelerating voltage of 80 kV. NW dimensions were determined by analyzing 10 TEM images of NWs with a total of more than 100 individual NWs of which length and width were measured. The dimensions thus determined are reported as average values $\pm$ standard error.

**Atomic Force Microscopy (AFM).** Atomic force microscopy was carried out on a NanoWizard II (JPK Instruments) microscope. 10 $\mu$L of dilute aqueous NW dispersions (0.1 mg/mL) were deposited onto freshly cleaved mica (SPI Supplies Division of Structure Probe, Inc.) and allowed to dry. The scans were performed in tapping mode in air using silicon cantilevers (NANO WORLD, TESPA-50) with a scan rate of 1 line/sec.

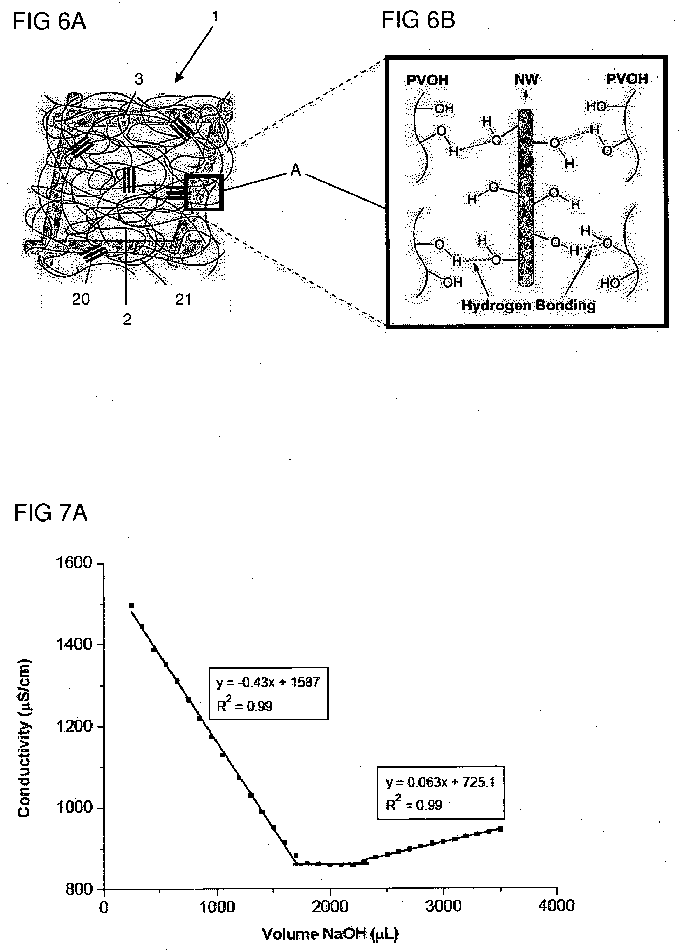

**Conductometric Titration.** Conductometric titrations were performed to quantify the surface charges of NWs. 50 mg of the NWs were suspended into 10-15 mL of aqueous 0.01 M hydrochloric acid. After 5 min of stirring and 30 min of sonication, the suspensions were titrated with 0.01 M NaOH. The titration curves show the presence of a strong acid, corresponding to the excess of HCl, and a weak acid corresponding to the sulfate-ester surface groups (see Fig. 7A to 7C).

**Swelling Behavior.** Prior to mechanical testing of ACSF-swollen samples, the degree of swelling was determined by measuring the weight of the samples pre- and post-swelling:

$$\text{Degree of swelling (\%)} = \frac{\text{Mass of wet sample - Mass of dry sample}}{\text{Mass of dry sample}} \times 100 \tag{1}$$

To minimize the error in measuring the degree of swelling, once the wet samples were taken out of the ACSF, they were placed on paper tissue to wick any excess ACSF from the surface; the samples were then immediately weighed.

**Dynamic Mechanical Analysis (DMA).** Mechanical properties of the PVOH/NW nanocomposites were characterized by dynamic mechanical analysis (DMA) using a TA instruments Model Q800. Tests were conducted in tensile mode using a temperature sweep method (0-140 °C) at a fixed frequency of 1 Hz, a strain amplitude of 30 $\mu$m, a heating rate of 5 °C/min, and a gap distance between jaws of ~10 mm. The samples were prepared by cutting strips from the films with a width of ~6 mm. To determine the mechanical properties of the films in the wet state, the samples were swelled in ACSF at 37 °C for periods of 1 week and 1 month. After the degree of swelling had been measured, DMA experiments were conducted in tensile mode with a submersion clamp, which allowed measurements while the samples were immersed in ACSF. In this case, the temperature sweeps were done in the range of 23-75 °C with a heating rate of 1 °C/min, a constant frequency of 1 Hz, a strain amplitude of 30 $\mu$m, and a fixed gap distance between jaws of 15 mm.

**Differential Scanning Calorimetry (DSC).** Differential scanning calorimetry experiments were carried out with a Mettler Toledo STAR instrument under N2 atmosphere. The typical procedure included heating and cooling cycles of approximately 10 mg sample in a DSC pan from -50 to 250 °C using a heating rate of 10 °C/min. The glass transition temperature ($T_g$) was determined from the midpoint of the specific heat increment at the glass-rubber transition, while the melting temperature ($T_m$) was taken as the peak temperature of the melting endotherm.

## RESULTS AND DISCUSSION

### Isolation and Physical Properties of Cellulose Nanowhiskers

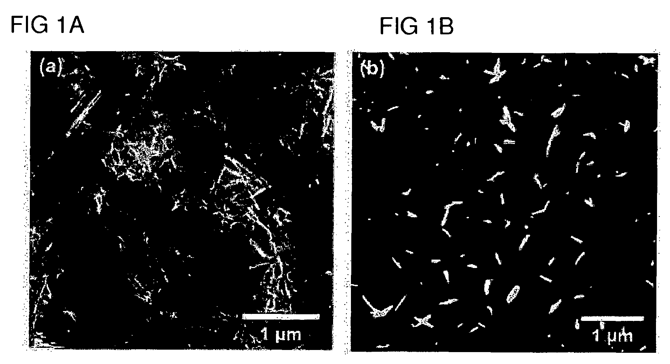

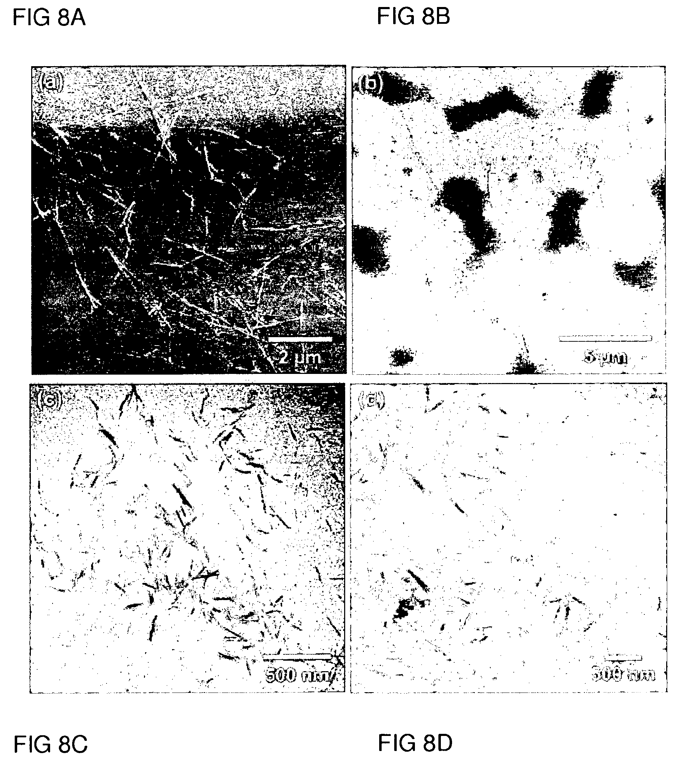

The NWs used in this study were isolated from tunicates (TNWs) and cotton (CNWs) by sulfuric acid hydrolysis, using protocols that represent modified versions of well-established methods. In the case of CNWs, spray-drying was used to isolate the dry NWs (see Experimental Section). Polymer nanocomposites with TNWs have consistently been shown to exhibit superior mechanical properties than those with CNWs, a fact that is mainly credited to their higher aspect ratio (~70 vs. ~10) and on-axis stiffness (tensile modulus ~143 vs. ~105 GPa). CNWs, on the other hand, are more viable for commercial exploitation because they are isolated from an abundant and sustainable bio-source. Due to their high density of strongly interacting surface hydroxyl groups, NWs have a strong tendency for self-association.30,43,46 Atomic force and electron microscopy of the NWs confirm that re-dispersion of the dried materials in water is readily possible (Fig. 1, and Fig. 8A to 8D). The dimensions of the TNWs, determined from TEM micrographs, were an average length and width of 2500 $\pm$ 1000 nm and 30 $\pm$ 5 nm, respectively. The average aspect ratio (A, defined as length to diameter (width) ratio, l/w) of the TNWs is therefore 83. The charge density of negatively charged sulphate esters on the NW surface that are introduced during hydrolysis has been suggested to modulate NW-NW interactions and to affect their dispersability. By conductometric titration, the density of sulfate groups of the present TNWs was determined to be ~90 mmol/kg (Fig. 7A).

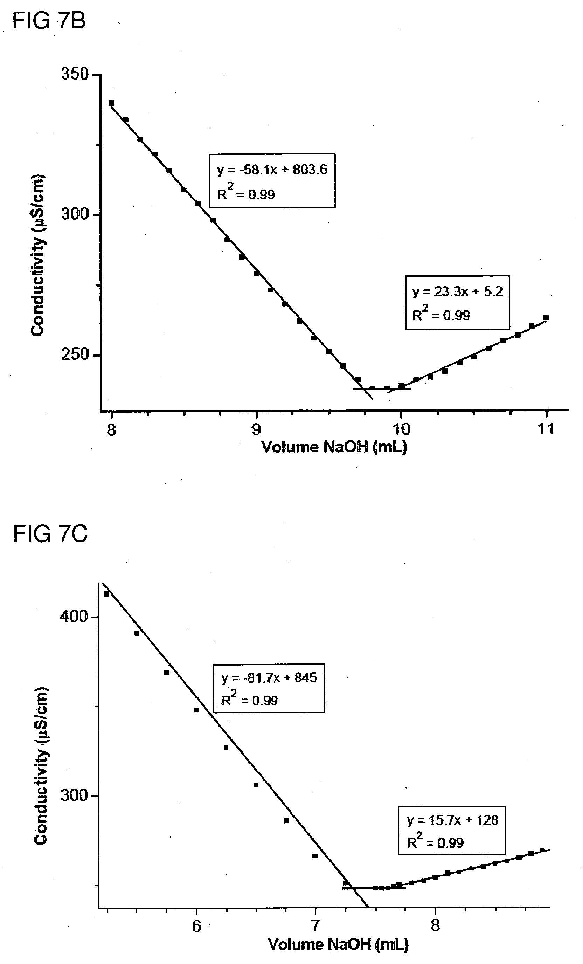

The CNWs used here were measured to have an average length and width of 220 $\pm$ 70 nm and 22 $\pm$ 6 nm, respectively, resulting in an aspect ratio of about 10. In addition to exhibiting a lower aspect ratio than the TNWs, the charge density on the surface of CNWs (~40 mmol/kg, see Fig. 7B and 7C), is significantly lower than that of TNWs. While TNWs were dried and isolated by lyophilization, spray-drying was used for CNWs. In order to assess any influence of the drying process on the physical properties of the CNWs, one batch of as-prepared CNWs was, after dialysis and sonication, split into two portions, which were dried by lyophilization and spray drying, respectively. TEM and conductometric titration data suggest that the drying method has no influence on the physical dimensions of the CNWs or on their surface charge density and morphology (Fig. 7A to 7C and Fig. 8A to 8D).

### Nanocomposite Processing

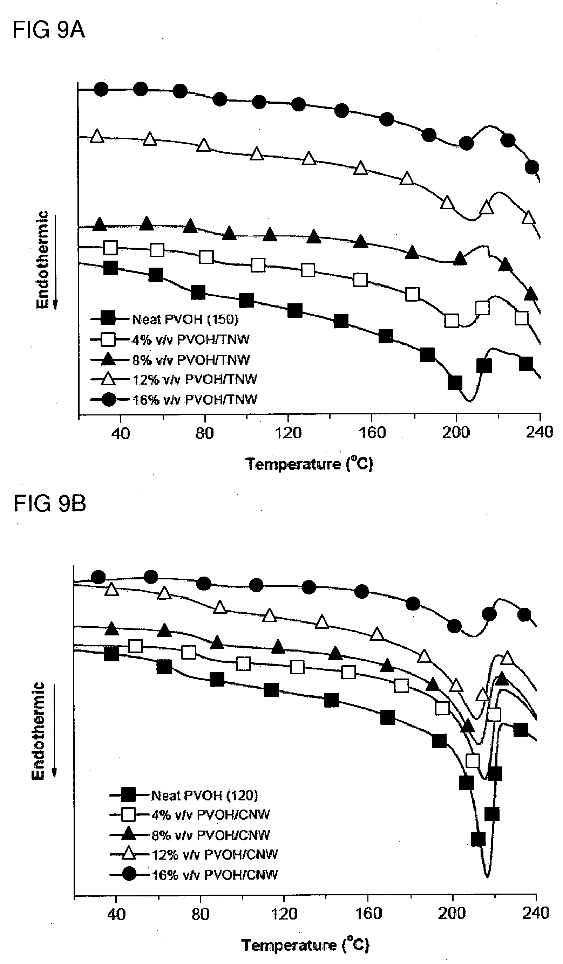

PVOH solutions and NW dispersions were combined, and after solution-casting and evaporation of solvent, the resulting films were re-shaped by compression-molding to result films of the nanocomposite with 4-16% v/v NWs and a thickness of 70-100 $\mu$m. Due to the limited thermal stability of the nanocomposites above the melting temperature ($T_m$) of PVOH (~220 °C), the films were compressed at a temperature much below $T_m$. PVOH/TNW nanocomposites were compression-molded at 150 °C without any visible color changes, while PVOH/CNW nanocomposites yellowed, when processed at this temperature (Fig. 9A, 9B). As a consequence, PVOH/CNW nanocomposites were processed at 120 °C, unless otherwise noted. Indeed, several explanations have been proposed in the literature regarding the degradation of NWs. The differences in thermal degradation may possibly arise from a combination of effects that including differences in surface chemistry and charge density, which are related to the source of NWs.

### Thermal Properties

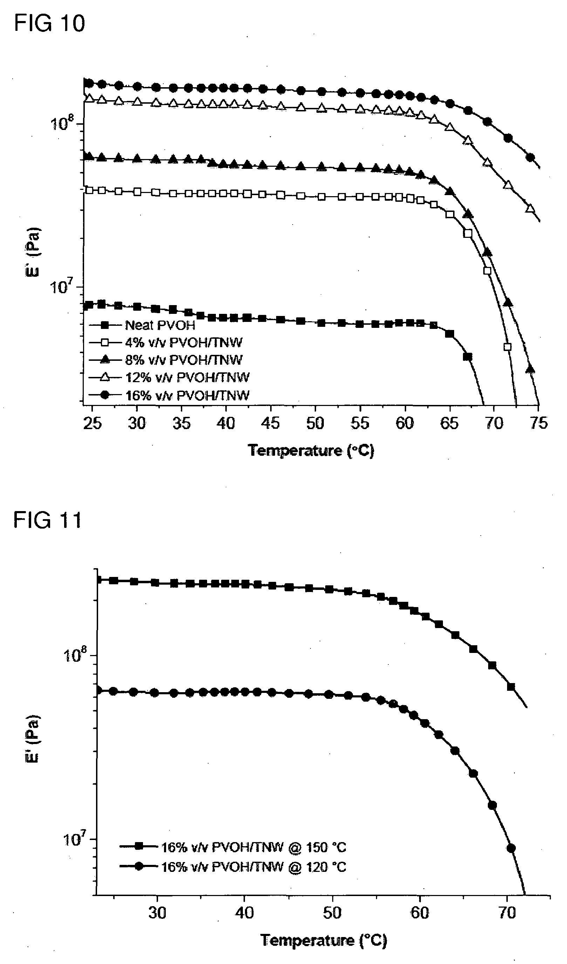

The thermal properties of PVOH/NW nanocomposites were determined using differential scanning calorimetry (DSC, Table 1). The DSC curves (Fig. 10) show that the $T_g$ (68 and 71 °C) and $T_m$ (216 and 206 °C) of the neat PVOH only slightly depends on the temperature at which the films were compression-molded. In both cases, the incorporation of NWs led to an increase of $T_g$ by approximately 10 °C, which interestingly was independent of the NW content. Upon introduction of NWs the width of the melting peak increases, and the degree of crystallinity ($\chi_c$) increases slightly, perhaps on account of a small nucleation effect of the NWs. Also this effect was largely independent of the NW content.

### Mechanical Properties of Dry PVOH/NW Nanocomposites

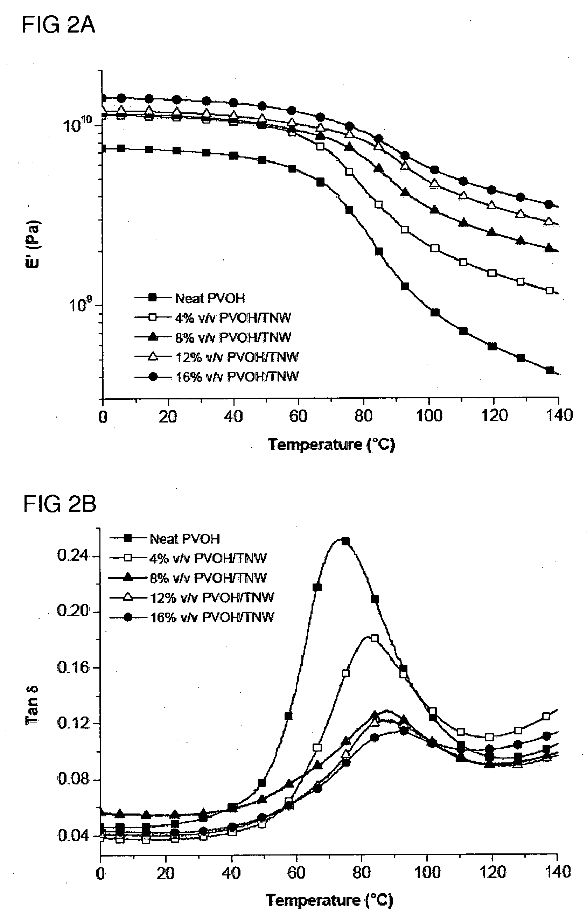

The mechanical properties of the nanocomposites were established using dynamic mechanical analysis (DMA, Table 2). Fig. 2A shows the tensile storage moduli (E') of the PVOH/TNW nanocomposites and a neat PVOH reference film in the dry state as a function of temperature. At room temperature (25 °C), the neat PVOH matrix, processed at 150 °C exhibits an E' of 7.3 GPa. Upon increasing the temperature, E' drastically decreases to 840 MPa at 100 °C ($\sim T_g$ + 30 °C) due to a transition from the glassy to the rubbery regime at -70 °C, which is seen as a maximum in the tan $\delta$ curves (Fig. 2B). PVOH/TNW nanocomposites containing 4 to 16 % v/v TNWs showed a significant increase in E' compared to the neat matrix below and above the $T_g$. At 25 °C, E' increased from 7.3 GPa (neat PVOH) to 13.7 GPa for the nanocomposite containing 16% v/v TNWs (Fig. 2A and Table 2). A more significant reinforcement was observed above $T_g$. At 100 °C, the nanocomposite containing 16% v/v TNWs shows an E' of 5.4 GPa, which represents a seven-fold increase over the stiffness of the neat PVOH (840 MPa) at this temperature.

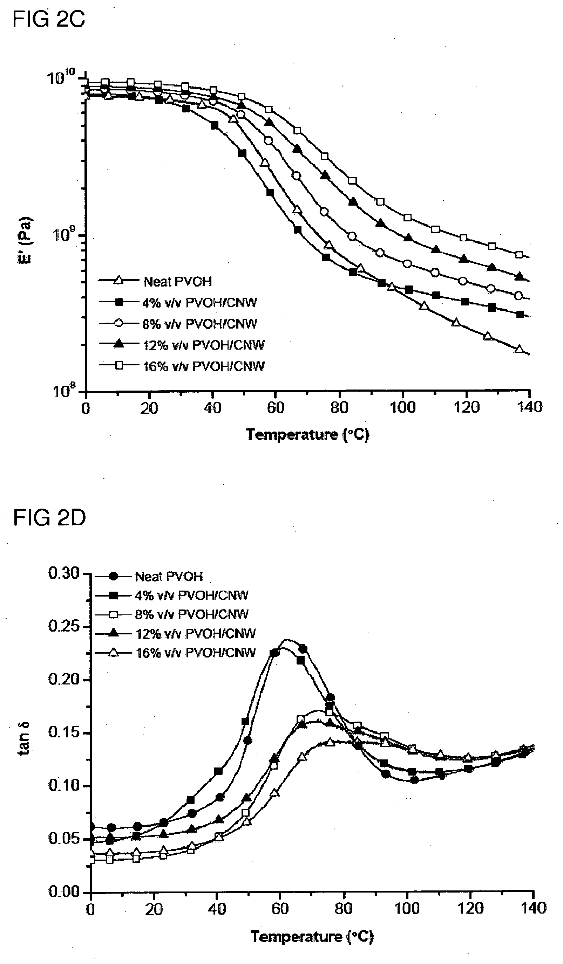

The E' of nanocomposites prepared with CNWs exhibits a similar trend as observed for the TNW nanocomposites, although the stiffness increase was more modest. As discussed above, PVOH/CNW nanocomposite films yellowed upon compression molding at 150 °C and were thus processed at 120 °C. At all temperatures, the E' of dry PVOH reference films, processed at 120 °C, was found to be slightly lower than that of the neat PVOH processed at 150 °C (Fig. 2A, 2C, and Table 2). For example, at 25 °C E' values of 7.3 and 7.0 GPa were measured. PVOH/CNW nanocomposite with 16% v/v CNWs exhibited a E' of 9.0 GPa at 25 °C, which is higher than that of the neat PVOH (7.0 GPa), but lower than the E' of 13.7 GPa of the TNW nanocomposite with the same NW content. Above $T_g$ (at 100 °C) E' of this nanocomposite was 1.4 GPa, which represent a two-fold enhancement over the stiffness of the neat polymer (Fig. 2C and Table 2). The lower reinforcement displayed by the CNWs compared to the TNWs is consistent with previous findings and is attributed at least in part to lower aspect ratio and stiffness of CNWs.

Fig. 2B and 2D display the loss factor (tan $\delta$) curves of neat PVOH and the two series of nanocomposites as a function of temperature. Tan $\delta$ is the ratio of loss modulus to storage modulus E"/E' of the material and is indicative of its damping behavior. All curves show a single relaxation peak centered at $T_\alpha$, which corresponds to the $T_g$ determined by DSC. The introduction of NWs led to a reduction in peak intensity and a shift of $T_\alpha$ to higher temperature compared to the neat PVOH films. This trend is attributed to the reduced mobility of PVOH chains in the amorphous phase due to the presence of the NWs.

Table 3 shows a comparison of the E' values of the dry PVOH/NW nanocomposites studied here with previously reported mechanically-adaptive nanocomposites based on a range of polymer matrices. The data are quoted for a filler content of $\sim$16% v/v. The comparison shows that the stiffness of the present PVOH/TNW nanocomposites is, at 25 °C as well as $T_g$ + 30 °C, more than three times higher than that of the stiffest mechanically-adaptive nanocomposites reported to date. This implies that a good dispersion of the NWs has been achieved in the PVOH matrix and supports the conclusion that strong H-bonding interactions between the NWs and the polymer matrix indeed increase the reinforcing effect of the cellulose.

This is illustrated in Fig. 6A and 6B. As shown in Fig. 6A, a polymer nanocomposite 1 comprises a matrix polymer 2, in the particular embodiment described herein PVOH, and a nanofiber network formed by a substantially three-dimensional network of nanofibers 3, in the particular embodiment described herein tunicate nanowhiskers (TNWs) or cotton nanowhiskers (CNWs). The matrix polymer comprises crystalline regions 20 and amorphous regions 21. As shown in Fig. 6B, the matrix polymer 2, in this case PVOH, is capable of forming hydrogen bonds with the nanofibers 3 (cellulose nanowhiskers NWs, in this case tunicate nanowhiskers TNWs or cotton nanowhiskers CNWs). Such hydrogen bonding (H-bonding) interactions between the NWs and the matrix polymer 2 are believed to have a reinforcing effect of the polymer nanocomposite 1 at least in the dry state of the polymer nanocomposite 1 (first switching state). Upon subjection to a stimulus, for example upon exposure to a water-containing composition, such hydrogen bonds at least partially are released, yielding a reduction in stiffness in the soft state (second switching state) of the nanocomposite 1.

The mechanical reinforcement in optimally assembled NW nanocomposites is caused by the formation of a percolating NW network, in which stress transfer is facilitated by hydrogen bonding between the NWs. The stiffness of these materials can be described by a percolation model that has been successfully used to predict the mechanical behavior of heterogeneous materials, such as polymer blends and nanocomposites. Detailed information about the percolation model and its use for modeling NWs-based nanocomposites has been reported for example in Samir, M. A. et al., Biomacromolecules, 2005, 6, 612. Fig. 3A and 3B show the predictions for the two nanocomposites series made by the percolation model, along with experimentally determined values of dry PVOH/NW nanocomposites at 100 °C, i.e., at $\sim T_g$ + 30 °C. For the calculations, aspect ratios (A) of 83 and 10 (as determined by TEM) were used for TNWs and CNWs, respectively, and storage moduli $E'_s$ of 840 MPa (for TNW nanocomposites processed at 150 °C) and 700 MPa (for CNW nanocomposites processed at 120 °C) were employed for the neat polymer matrix at 100 °C (as determined by DMA). The tensile storage modulus of the NW phase, $E'_r$, was in previous studies derived by either measuring the stiffness of a neat TNW or CNW film or by fitting the model against the experimentally determined properties of the nanocomposites and using $E'_r$ as a fit parameter. While the morphology (and therewith the stiffness) of a neat NW film depends strongly on the processing conditions and has little resemblance to that of a NW network within a polymer matrix, the $E'_r$ values of 5 - 24 GPa for TNW-based, and 0.6 - 5 GPa for CNW-based nanocomposites determined by these approaches appeared to roughly match. Interestingly, $E'_r$ values of 80 GPa and 10 GPa are required to fit the model to the data for the TNW-based and CNW-based nanocomposites with PVOH studied here (Fig. 3A and 3B). A comparison of the data for several other TNW-based nanocomposites shows that for a given NW content, E' increases with the polarity of the polymer matrix (PS<PVAc<Epoxy), suggesting that systems with pronounced NW-polymer interactions may exhibit larger reinforcement due to factors that are not explicitly accounted for in the percolation model.

**Swelling Behavior**

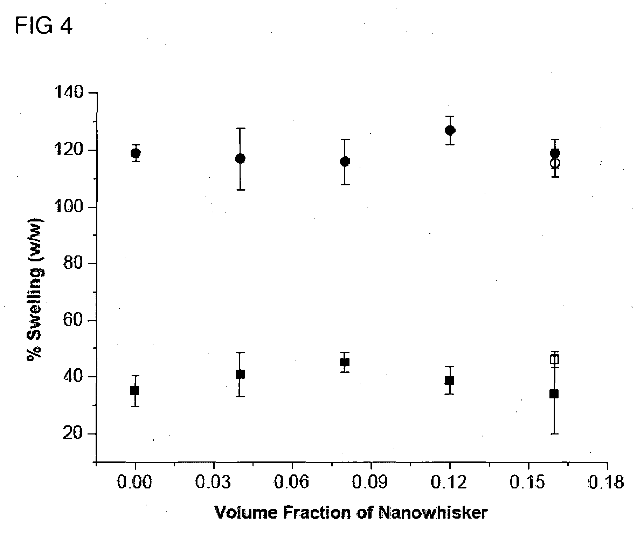

The swelling behavior of the nanocomposites in physiological conditions was investigated by immersing the materials into artificial cerebrospinal fluid (ACSF) at 37 °C to mimic physiological conditions. It is known that heat-treated PVOH is no longer water soluble, and that the processing temperature of PVOH affects the permeability of the material and thereby the potential for water uptake. Indeed, the swelling characteristics of the materials studied here were found to be strongly dependent on the temperature used for compression molding, but not the type or content of NWs. Neat PVOH and PVOH/TNW nanocomposite films processed at 150 °C exhibit ~40% w/w swelling, whereas neat PVOH films and PVOH/CNW nanocomposites processed at 120 °C exhibit approximately ~120% w/w swelling (Fig. 4), regardless of the NW content. The conclusion that the processing temperature is the primary factor for the swelling behavior was further confirmed by swelling PVOH/CNW nanocomposites, which were processed at 150 °C, and PVOH/TNW nanocomposites, which were processed at 120 °C (Fig. 4). Compared to the other nanocomposites, these samples exhibited swelling that was consistent with their processing temperature rather than the type of NW. Swelling experiments in ACSF were extended over the course of two months to investigate the possible changes that might occur during prolonged biological implantation of the material (Table 4). The data show that for the PVOH/TNW nanocomposites processed at 150 °C equilibrium swelling is reached within 24 hours and that these materials do not degrade over the course of two months. Similarly, PVOH/CNW nanocomposite films reached an equilibrated swelling within 24 hours and maintained their integrity for at least one week. A comparison of the swelling data of the present PVOH/TNW and the previously investigated PVAc/TNW nanocomposites29 shows that the PVOH-based nanocomposites swell much less than their PVAc-based counterparts. For instance, the PVAc/TNW nanocomposites comprising 16.5% v/v TNWs displayed a degree of swelling of ~80%, while the PVOH/TNW nanocomposites shows ~40% w/w of swelling with 16% v/v NWs.

**Mechanical Properties of ACSF-Swollen Nanocomposites**

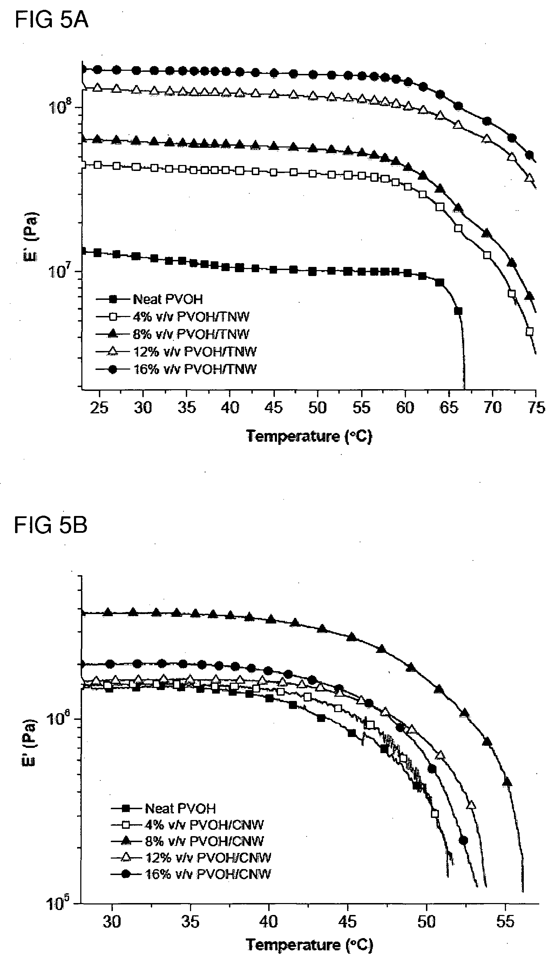

The mechanical properties of ACSF-swollen PVOH/NW nanocomposites were determined by DMA using a submersion clamp set-up, which allowed the samples to be immersed in ACSF during the measurements. Neat PVOH films softened substantially upon submersion in ACSF for one week and exhibited mechanical properties that appear to be correlated with their swelling behavior. Neat PVOH films processed at 150 °C displayed a change in E' from ~7.3 GPa (dry) to ~11 MPa (ACSF-swollen), whereas E' for neat PVOH films processed at 120 °C changed from 7.0 GPa to ~1 MPa. The ACSF-swollen PVOH/TNW nanocomposites display an E' that is higher than of the neat PVOH films (Fig. 5 and Fig. 10), but considerably lower than that of the corresponding materials in the dry state. For example, E' of the material comprising 16% v/v TNWs dropped from 13.7 GPa (dry, RT) to ~160 MPa (ACSF-swollen at 37 °C). The data in Fig. 3 and Table 2 show that the switching is reversible and that ACSF exposure for 1 week and 1 month has the same effect. Moreover, the data in Fig. 3A show that the relation between E' of the ACSF-swollen PVOH/TNW nanocomposites and the TNW content is fairly well described by the Halpin-Kardos model, whose application to the modeling of NWs-based nanocomposites has been reported for example by Shanmuganathan, K. et al., J. Mater. Chem., 2010, 20, 180. Parameters used for the modeling were taken from Hajji, P. et al., Polym. Compos., 1996, 17, 612, except $E'_s$ of 11 MPa and 1.44 MPa for materials processed at 150 and 120 °C, respectively, which were measured by DMA. The fact that the model underestimates E' suggests that the NW-NW and perhaps also NW-matrix interactions are reduced upon swelling with ACSF, but - perhaps on account to the abundance of hydroxyl groups on the matrix polymer and the NWs that can interact with water - not entirely switched off. All of the ACSF-swollen samples show a significant drop of E' at -60 °C. Since this temperature is below the $T_g$ of the plasticized PVOH and far below the $T_m$ of the matrix (~220 °C), this transition may relate to the dissolution of the matrix.

The ACSF-swollen PVOH and PVOH/CNW films, which were processed at 120 °C and display considerably more swelling than the above-discussed TNW-based materials, exhibit an E' of 1 - 4 MPa at 37 °C (Fig. 3B and 5B, Table 2). Due to the rather low modulus, these measurements feature a significant error, and it is therefore not possible to draw a firm conclusion about how the nanocomposite composition affects the modulus of the ACSF-swollen materials. The fact that the ACSF-induced mechanical switching was very significant and the finding that similar values were observed for both the neat PVOH and the nanocomposites suggest that the mechanical properties of these materials were largely governed by the swollen polymer matrix, supporting the notion that the water effectively reduced hydrogen bonding between the NWs.

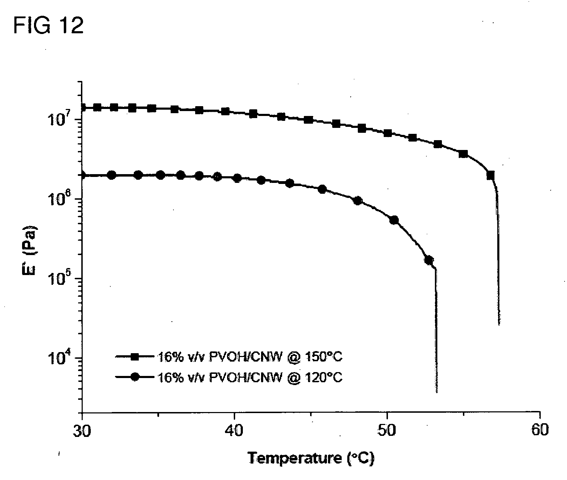

In order to further probe the influence of the processing temperature on the mechanical switching, the tensile storage moduli (E') of nanocomposites with 16% v/v TNWs that had been compression-molded at 120 °C and 150 °C were compared (Table 2). It was found that the E' of PVOH/TNW films processed at 150 °C, switched from 13.7 GPa (dry, RT) to 164 MPa (ACSF-swollen at 37 °C), whereas the material processed at 120 °C switched from 12.3 GPa to 60 MPa (Fig. 11). A similar result, was observed for the ACSF-swollen PVOH/CNW films, which if processed at 150 °C exhibited an E' of 13 MPa at 37 °C, while the same composition processed at 120 °C, showed an E' of 2 MPa at 37 °C (Fig. 12). A lower processing temperature therefore not only influences the mechanical properties of the materials in the dry state, but also reduces E' of the ACSF-swollen state considerably. While the PVOH/CNW films processed at 150 °C show some yellowing, their dry-state mechanical properties are largely comparable to those of the material processed at 120 °C, and (on account of less swelling) a higher modulus in the soft state is achieved, suggesting that whatever effect is responsible for the yellowing, it is not negatively impacting the material's mechanical characteristics. Thus, it appears that if PVOH is used as a matrix, not only the NW type and content but also the processing temperature can be used to tailor the mechanical contrast of cellulose NWs-based, water-responsive, mechanically adaptive nanocomposites.

In summary of the experimental results and discussion given above, water-responsive, mechanically-adaptive nanocomposites based on polymer nanocomposites described herein, in particular such nanocomposites using PVOH as matrix polymer and TNWs or CNWs as nanofibers, may offer an initial stiffness that is significantly higher than that of previous generations of such responsive materials.

In particular the use of PVOH as a matrix polymer into which nanofibers such as NWs are incorporated may yield tensile storage moduli of PVOH/NW nanocomposites which - in both the glassy and rubbery regime - in the first switching state are significantly higher than those of comparable, other nanocomposites. It appears that in addition to NW-NW interactions, polymer-NW interactions, which are promoted by the strong propensity of PVOH to form hydrogen bonds, may be a factor in this context. Another factor is the possibility of controlling the swelling characteristics of the PVOH matrix, and therewith the properties of water- or ACSF-swollen nanocomposites, via the processing conditions.

By such means, the change in stiffness upon switching between the switching states of the TNW-based nanocomposites upon exposure to ACSF could be varied between a 90-fold to a 200-fold modulus change. By fine-tuning the process conditions a desired modulus change upon exposure to a suitable stimulus may be set.

Although not as stiff initially in the dry state compared to PVOH/TNW, PVOH/CNW nanocomposites exhibit a larger mechanical contrast (up to 900-fold), as they soften more than TNW-based nanocomposites. This effect possibly is related to the lower reinforcing power of CNWs. Despite the fact that decomposition of the CNW-containing materials starts around 150 °C, the nanocomposites maintain useful mechanical properties. Therefore, one could envision employing a higher processing temperature for PVOH/CNW nanocomposites to further increase the stiffness of the water-swollen state.

The invention is not limited to the embodiments described above but may be carried out also by entirely different embodiments. In particular, other matrix polymer materials such as polyamide may be used rather than PVOH. Further, different nanofibers other than tunicate nanowhiskers (TNW) or cotton nanowhiskers (CNW), for example cellulose nanowhiskers not derived from tunicates or cotton, may be employed.

## List of Reference Numerals

| | |
|---|---|
| 1 | Composite |
| 2 | Matrix polymer |
| 20 | Crystalline region |
| 21 | Amorphous region |
| 3 | Nanofibers (cellulose nanowhiskers) |
| 4 | Medical injection device |
| 40 | Hollow tube section |
| 400 | Inner bore |
| 41 | Tip |
| 42 | Opening |
| 5 | Skin |
| $\alpha$ | Angle |
| E | Cutting plane |
| L | Direction |

Table 1

| Sample | NW Content (% v/v) | $T_g$ (°C)[a] | $T_m$ (°C)[a] | $\Delta H_m$ (J/g) | $\chi_c$ (%)[b] |
|---|---|---|---|---|---|
| Neat PVOH[c] | | 71 | 206 | 43.9 | 27 |

(continued)

| Sample | NW Content (% v/v) | $T_g$ (°C)[a] | $T_m$ (°C)[a] | $\Delta H_m$ (J/g) | $\chi_c$ (%)[b] |
|---|---|---|---|---|---|
| PVOH/TNW | 4 | 81 | 204 | 50.2 | 33 |
|  | 8 | 83 | 196 | 49.7 | 34 |
|  | 12 | 82 | 208 | 48.8 | 35 |
|  | 16 | 82 | 201 | 44.1 | 34 |
| Neat PVOH[d] |  | 68 | 216 | 49.6 | 31 |
| PVOH/CNW | 4 | 79 | 216 | 58.5 | 38 |
|  | 8 | 80 | 213 | 52.4 | 36 |
|  | 12 | 81 | 212 | 47.3 | 34 |
|  | 16 | 82 | 210 | 40.9 | 31 |

[a]The data obtained from the second heating scan of DSC measurements. [b]$\chi_c = \frac{\Delta H_m}{w \Delta H_0}$, were $w$ is the weight fraction of polymer matrix in the nanocomposites, $\Delta H_m$ is the measured melting enthalpy and $\Delta H_o$ is the enthalpy of 100% aystalline PVOH (161 J/g).[58] [c]Films were compression-molded at 150 °C. [d] Films were compression-molded at 120 °C.

Table 2

| Sample | NW Content (% v/v) | Dry Nanocomposites | | ACSF-swollen Nanocomposites | |
|---|---|---|---|---|---|
|  |  | E' at 25°C (GPa) | E' at 100 °C (GPa) | E' at 37 °C after 1 week in ACSF (MPa) | E' at 37 °C after 1 month in ACSF (MPa) |
| Neat PVOH[a] |  | 7.3 | 0.84 | 11.1 | 6.9 |
| PVOH/TNW | 4 | 10.5 | 2.1 | 45.4 | 46.8 |
|  | 8 | 11.1 | 3.7 | 78.3 | 85.2 |
|  | 12 | 11.7 | 4.7 | 124 | 108 |
|  | 16 | 13.7 | 5.4 | 164 | 173 |
|  | 16[b] | 1.2.3 | 3.0 | 60[c] | n.m. |
| Neat PVOH[b] |  | 7.0 | 0.7 | 1.4[c] | n.m. |
| PVOH/CNW | 4 | 6.8 | 0.5 | 1.5[c] | n.m. |
|  | 8 | 7.7 | 0.8 | 3.6[c] | n.m. |
|  | 12 | 8.3 | 1.0 | 1.6[c] | n.m. |
|  | 16 | 9.0 | 1.4 | 1.9[c] | n.m. |
|  | 16[a] | 8.4 | 2.1 | 13[c] | n.m. |

[a]Films were compression-molded at 150 °C. [b]Films were compression-molded at 120 °C. [c]Samples were measured after 1 day immersed in ACSF nm = not measured.

Table 3

| NW Type | Polymer Matrix | E' of Neat Polymer at 25°C (GPa) | E' of Nanocomposite at 25 °C (GPa) | E' of Nanocomposite at $T_g$ + 30 °C (GPa) |
|---|---|---|---|---|
| Tunicate | EO-EPI | 0.004 | 0.8 | n.a. |
| Tunicate | PBMA | 0.6 | 3.8 | 1.1 |
| Tunicate | PVAc | 2.0 | 5.2 | .7 |
| Cotton | PVAc | 2.0 | 4.0 | .05 |
| Tunicate | PVOH | 7.3[a] | 13.7 | 5.4 |
| Cotton | PVOH | 7.0[b] | 9.0 | 1.4 |

[a]PVOH film was compression-moided at 150 °C. [b]PVOH film was compression-molded at 120 °C.

Table 4

| Sample | TNW Content (% v/v) | Alter 1 Day (% w/w) | After 3 Days (% w/w) | After 1 Week (% w/w) | After 1 Month (% w/w) | After 2 Months(% w/w) |
|---|---|---|---|---|---|---|
| Neat PVOH | | 35 ± 5 | 43 ± 4 | 38 ± 2 | 43 ± 4 | 45 ± 6 |
| PVOH/TNW | 4 | 41 ± 8 | 40 ± 6 | 48 ± 2 | 37 ± 8 | 50 ± 3 |
| | 8 | 45 ± 3 | 44 ± 7 | 42 ± 5 | 47 ± 11 | 48 ± 6 |
| | 12 | 39 ± 5 | 46 ± 9 | 37 ± 5 | 36 ± 5 | 46 ± 8 |
| | 16 | 34 ± 1 | 42 ± 3 | 40 ± 8 | 42 ± 5 | 41 ± 2 |

**Claims**

1. A medical injection device (4) for guiding liquid having switchable mechanical properties, comprising a hollow tube section (40) having an inner bore (400), wherein the hollow tube section (40) at least partially is made of a material exhibiting switchable mechanical properties such that the material in a first switching state comprises a first stiffness **characterized by** a first tensile storage modulus (E'), in a second switching state comprises a second stiffness **characterized by** a second tensile storage modulus (E') smaller than the first tensile storage modulus and is switchable from the first switching state to the second switching state by exposing the material to a stimulus, **characterized in that** said material comprises a polymer nanocomposite (1) comprising

    - a matrix polymer (2) and
    - a nanoparticle network, wherein the nanoparticle network is formed by a formation of a substantially three-dimensional network of nanoparticles (3) which are incorporated in the matrix polymer (2) and interact with each other and/or with the matrix polymer (2),

    wherein said nanoparticles (3) comprise nanofibers, and
    wherein said stimulus influences interactions among the nanoparticles (3) and/or between the nanoparticles (3) and the matrix polymer (2).

2. The medical injection device (4) of claim 1, wherein the first tensile storage modulus (E') is greater than 3.5 GPa, preferably 6 GPa, most preferably larger than 9 GPa, and/or wherein the second tensile storage modulus (E') is less than 1 GPa.

3. The medical injection device (4) of one of claims 1 to 2, wherein the polymer nanocomposite (1) is obtainable by applying a process comprising the steps of:

    - providing the matrix polymer (2),
    - incorporating the nanoparticle network into the matrix polymer (2) to obtain an intermediate mixture,
    - subjecting the intermediate mixture to a heat treatment step involving exposure to a temperature equal to or greater than 100 °C.

4. The medical injection device (4) of claim 3, wherein the heat treatment step includes compression molding the polymer nanocomposite (1) at a temperature equal to or greater than 100 °C.

5. The medical injection device (4) of claim 4 or 5,
    wherein, prior to the heat treatment step, the intermediate mixture is subjected to a drying step involving exposure to an elevated temperature of 30 °C to 80 °C over an elongated period of time of 1 day to 10 days and/or
    wherein the drying step includes

    - a first drying step including exposure to a first drying temperature of 30 °C to 50 °C, for example 35 °C, over a first period of time of 1 day to 10 days, for example 5 days, and a
    - second drying step including exposure to a second drying temperature of 50 °C to 80 C, for example 70 °C, over a second period of time of 5h to 48h, for example 24h.

6.  The medical injection device (4) of one of the preceding claims, wherein the matrix polymer (2) comprises a polymer capable of forming non-covalent interactions, in particular hydrogen bonds, with the nanoparticles (3).

7.  The medical injection device (4) of one of the preceding claims, wherein said matrix polymer (2) comprises at least one of vinyl polymer such as polyvinyl alcohol, poly(acrylic acid), poly(acryl amide), polyvinyl pyridine), a copolymer of at least one of such monomers, a polycondensate such as polyamide or polyester, a polysaccharide such as cellulose, starch, alginates, pectins, hyaluronane, chitin, chitosan or at least one of its derivatives, and/or a protein.

8.  The medical injection device (4) of one of the preceding claims, wherein the nanoparticles (3) are capable of forming non-covalent interactions, in particular hydrogen bonds, with each other and/or with the matrix polymer (2).

9.  The medical injection device (4) of claim 9, wherein the nanofibers (3) comprise cellulose nanowhiskers, preferably wherein the nanofibers (3) comprise cellulose nanowhiskers derived from tunicates or cotton.

10. The medical injection device (4) of one of claims 9 to 10, wherein the nanofibers (3) comprise an aspect ratio greater than 5, preferably greater than 10, more preferably greater than 50, most preferably greater than 80.

11. The medical injection device (4) of one of the preceding claims, wherein the polymer nanocomposite (1) comprises a content of 1 % to 30 %, preferably 2 % to 25 %, more preferably 3 % to 20 % v/v of nanoparticles (3).

12. The medical injection device (4) of one of the preceding claims, wherein the polymer nanocomposite (1) is switchable from the first switching state to the second switching state by a stimulus which reduces the interactions among the nanoparticles (3) and/or between the nanoparticles (3) and the matrix polymer (2), and the polymer nanocomposite (1) is switchable from the second switching state to the first switching state by a stimulus which enhances the interactions among the nanoparticles (3) and/or between the nanoparticles (3) and the matrix polymer (2).

13. The medical injection device (4) of one of the preceding claims,
    wherein the polymer nanocomposite (1) is switchable from the first switching state to the second switching state by subjecting the polymer nanocomposite (1) to a water-containing composition and/or
    wherein the polymer nanocomposite (1) is switchable by subjecting the polymer nanocomposite (1) to a composition at an elevated temperature between 30 °C and 50°C, preferably 35 °C to 40 °C.

14. The medical injection device (4) of one of the preceding claims,
    wherein the ratio of the storage moduli of the two switching states is more than 20, preferably more than 50, more preferably more than 100, and most preferably more than 500 and/or
    wherein the polymer nanocomposite (3) displays essentially isotropic mechanical properties and/or
    wherein the hollow tube section (40) has a wall thickness between 50 $\mu$m and 1000 $\mu$m, preferably between 100 $\mu$m to 500 $\mu$m.

15. A method for producing a medical injection device (4) having switchable mechanical properties, comprising a hollow tube section (40) having an inner bore (400), wherein the hollow tube section (40) at least partially is made of a material exhibiting switchable mechanical properties such that the material in a first switching state comprises a first stiffness **characterized by** a first tensile storage modulus (E'), in a second switching state comprises a second stiffness **characterized by** a second tensile storage modulus (E') smaller than the first tensile storage modulus and is switchable from the first switching state to the second switching state by exposing the material to a stimulus, **characterized in that** said material comprises a polymer nanocomposite which is produced by

    - providing a matrix polymer (2) and
    - incorporating a nanoparticle network into the matrix polymer (2), wherein the nanoparticle network is formed by a formation of a substantially three-dimensional network of nanoparticles (3) which are incorporated in the matrix polymer (2) and interact with each other and/or with the matrix polymer (2), wherein said stimulus influences interactions among the nanoparticles (3) and/or between the nanoparticles (3) and the matrix polymer (2).

    wherein said nanoparticles (3) comprise nanofibers.

16. A method for inducing a stiffness change in a medical injection device (4) having switchable mechanical properties and comprising a hollow tube section (40) having an inner bore (400), wherein the hollow tube section (40) at least partially is made of a material exhibiting switchable mechanical properties such that the material in a first switching state comprises a first stiffness **characterized by** a first tensile storage modulus (E'), in a second switching state comprises a second stiffness **characterized by** a second tensile storage modulus (E') smaller than the first tensile storage modulus and is switched from the first switching state to the second switching state by exposing the material to a stimulus,

**characterized in**

**that** said material comprises a polymer nanocomposite comprising

- a matrix polymer (2) and
- a nanoparticle network, wherein the nanoparticle network is formed by a formation of a substantially three-dimensional network of nanoparticles (3) which are incorporated in the matrix polymer (2) and interact with each other and/or with the matrix polymer (2),
wherein said stimulus switches the polymer nanocomposite between the first switching state and the second switching state by influencing interactions among the nanoparticles (3) and/or between the nanoparticles (3) and the matrix polymer (2),

wherein said nanoparticles (3) comprise nanofibers.

**Patentansprüche**

1. Medizinische Injektionsvorrichtung (4) zum Führen von Flüssigkeit mit umschaltbaren mechanischen Eigenschaften, umfassend einen hohlen Rohrabschnitt (40) mit einer Innenbohrung (400), wobei der hohle Rohrabschnitt (40) wenigstens teilweise aus einem Material besteht, das umschaltbare mechanische Eigenschaften aufweist, so dass das Material in einem ersten Schaltzustand eine erste Steifigkeit aufweist, die durch einen ersten Zug-Speichermodul (E') gekennzeichnet ist, in einem zweiten Schaltzustand eine zweite Steifigkeit aufweist, die durch einen zweiten Zug-Speichermodul (E') gekennzeichnet ist, der kleiner als der erste Zug-Speichermodul ist und von dem ersten Schaltzustand in den zweiten Schaltzustand umschaltbar ist, indem das Material gegenüber einem Reiz exponiert wird,

**dadurch gekennzeichnet,**

**dass** das Material ein Polymer-Nanokomposit (1) umfasst, umfassend

- ein Matrixpolymer (2) und
- ein Nanopartikelnetzwerk, wobei das Nanopartikelnetzwerk durch Bilden eines im Wesentlichen dreidimensionalen Netzwerks von Nanopartikeln (3) gebildet ist, die in das Matrixpolymer (2) einverleibt sind und miteinander und/oder mit dem Matrixpolymer (2) wechselwirken,

wobei die Nanopartikel (3) Nanofasern umfassen und
wobei der Reiz Wechselwirkungen der Nanopartikel (3) miteinander und/oder zwischen den Nanopartikeln (3) und dem Matrixpolymer (2) beeinflusst.

2. Medizinische Injektionsvorrichtung (4) gemäß Anspruch 1, wobei der erste Zug-Speichermodul (E') größer als 3,5 GPa ist, vorzugsweise 6 GPa, höchst bevorzugt größer als 9 GPa, und/oder wobei der zweite Zug-Speichermodul (E') kleiner als 1 GPa ist.

3. Medizinische Injektionsvorrichtung (4) gemäß einem der Ansprüche 1 bis 2, wobei das Polymer-Nanokomposit (1) durch Anwenden eines Verfahrens umfassend die Schritte:

- Bereitstellen des Matrixpolymers (2),
- Einverleiben des Nanopartikelnetzwerks in das Matrixpolymer (2), um ein intermediäres Gemisch zu erhalten,
- Unterwerfen des intermediären Gemischs an einen Wärmebehandlungsschritt, der Exposition gegenüber einer Temperatur von gleich oder höher als 100 °C umfasst,

erhältlich ist.

4. Medizinische Injektionsvorrichtung (4) gemäß Anspruch 3, wobei der Wärmebehandlungsschritt Formpressen des Polymer-Nanokomposits (1) bei einer Temperatur von gleich oder höher als 100 °C umfasst.

**5.** Medizinische Injektionsvorrichtung (4) gemäß Anspruch 4 oder 5,
wobei das intermediäre Gemisch vor dem Wärmebehandlungsschritt einem Trocknungsschritt unterworfen wird, der Exposition gegenüber einer erhöhten Temperatur von 30 °C bis 80 °C über einen ausgedehnten Zeitraum von 1 Tag bis 10 Tagen umfasst und/oder
wobei der Trocknungsschritt umfasst:

- einen ersten Trocknungsschritt, der Exposition gegenüber einer ersten Trocknungstemperatur von 30 °C bis 50 °C, beispielsweise 35 °C, über einen ersten Zeitraum von 1 Tag bis 10 Tagen, beispielsweise 5 Tagen, umfasst, und
- einen zweiten Trocknungsschritt, der Exposition gegenüber einer zweiten Trocknungstemperatur von 50 °C bis 80 °C, beispielsweise 70 °C, über einen zweiten Zeitraum von 5 h bis 48 h, beispielsweise 24 h, umfasst.

**6.** Medizinische Injektionsvorrichtung (4) gemäß einem der vorstehenden Ansprüche, wobei das Matrixpolymer (2) ein Polymer umfasst, das fähig ist, nichtkovalente Wechselwirkungen, insbesondere Wasserstoffbrücken, mit den Nanopartikeln (3) zu bilden.

**7.** Medizinische Injektionsvorrichtung (4) gemäß einem der vorstehenden Ansprüche, wobei das Matrixpolymer (2) wenigstens ein Vinylpolymer, wie z. B. Polyvinylalkohol, Poly(acrylsäure), Poly(acrylamid), Poly(vinylpyridin), ein Copolymer von wenigstens einem dieser Monomere, ein Polykondensat, wie z. B. Polyamid oder Polyester, ein Polysaccharid, wie z. B. Cellulose, Stärke, Alginate, Pectine, Hyaluronan, Chitin, Chitosan oder wenigstens eines seiner Derivate und/oder ein Protein umfasst.

**8.** Medizinische Injektionsvorrichtung (4) gemäß einem der vorstehenden Ansprüche, wobei die Nanopartikel (3) fähig sind, nichtkovalente Wechselwirkungen, insbesondere Wasserstoffbrücken, miteinander und/oder mit dem Matrixpolymer (2) zu bilden.

**9.** Medizinische Injektionsvorrichtung (4) gemäß Anspruch 9, wobei die Nanofasern (3) Cellulose-Nanowhisker umfassen, vorzugsweise wobei die Nanofasern (3) aus Tunicaten oder Baumwolle abgeleitete Cellulose-Nanowhisker umfassen.

**10.** Medizinische Injektionsvorrichtung (4) gemäß einem der Ansprüche 9 bis 10, wobei die Nanofasern (3) ein Aspektverhältnis von größer als 5 aufweisen, vorzugsweise größer als 10, bevorzugter größer als 50, höchst bevorzugt größer als 80.

**11.** Medizinische Injektionsvorrichtung (4) gemäß einem der vorstehenden Ansprüche, wobei das Polymer-Nanokomposit (1) einen Gehalt von 1 % bis 30 %, vorzugsweise 2 % bis 25 %, bevorzugter 3 % bis 20 % v/v an Nanopartikeln (3) umfasst.

**12.** Medizinische Injektionsvorrichtung (4) gemäß einem der vorstehenden Ansprüche, wobei das Polymer-Nanokomposit (1) durch einen Reiz, der die Wechselwirkungen der Nanopartikel (3) miteinander und/oder zwischen den Nanopartikeln (3) und dem Matrixpolymer (2) verringert, von dem ersten Schaltzustand in den zweiten Schaltzustand umschaltbar ist und das Polymer-Nanokomposit (1) durch einen Reiz, der die Wechselwirkungen der Nanopartikel (3) miteinander und/oder zwischen den Nanopartikeln (3) und dem Matrixpolymer (2) verstärkt, von dem zweiten Schaltzustand in den ersten Schaltzustand umschaltbar ist.

**13.** Medizinische Injektionsvorrichtung (4) gemäß einem der vorstehenden Ansprüche,
wobei das Polymer-Nanokomposit (1) von dem ersten Schaltzustand in den zweiten Schaltzustand umschaltbar ist, indem das Polymer-Nanokomposit (1) an eine wasserhaltige Zusammensetzung ausgesetzt wird und/oder
wobei das Polymer-Nanokomposit (1) umschaltbar ist, indem das Polymer-Nanokomposit (1) an eine erhöhte Temperatur zwischen 30 °C und 50 °C, vorzugsweise 35 °C bis 40 °C, ausgesetzt wird.

**14.** Medizinische Injektionsvorrichtung (4) gemäß einem der vorstehenden Ansprüche,
wobei das Verhältnis der Speichermodule der beiden Umschaltzustände höher als 20 ist, vorzugsweise höher als 50, bevorzugter höher als 100 und höchst bevorzugt höher als 500 und/oder
wobei das Polymer-Nanokomposit (3) im Wesentlichen isotrope mechanische Eigenschaften zeigt und/oder
wobei der hohle Rohrabschnitt (40) eine Wanddicke zwischen 50 $\mu$m und 1000 $\mu$m aufweist, vorzugsweise zwischen 100 $\mu$m und 500 $\mu$m.

**15.** Verfahren zum Herstellen einer medizinischen Injektionsvorrichtung (4) mit umschaltbaren mechanischen Eigenschaften, umfassend einen hohlen Rohrabschnitt (40) mit einer Innenbohrung (400), wobei der hohle Rohrabschnitt (40) wenigstens teilweise aus einem Material besteht, das umschaltbare mechanische Eigenschaften aufweist, so dass das Material in einem ersten Schaltzustand eine erste Steifigkeit aufweist, die durch einen ersten Zug-Speichermodul (E') gekennzeichnet ist, in einem zweiten Schaltzustand eine zweite Steifigkeit aufweist, die durch einen zweiten Zug-Speichermodul (E') gekennzeichnet ist, der kleiner als der erste Zug-Speichermodul ist und von dem ersten Schaltzustand in den zweiten Schaltzustand umschaltbar ist, indem das Material gegenüber einem Reiz exponiert wird,
**dadurch gekennzeichnet,**
**dass** das Material ein Polymer-Nanokomposit umfasst, das hergestellt wird durch

- Bereitstellen eines Matrixpolymers (2) und
- Einverleiben eines Nanopartikelnetzwerks in das Matrixpolymer (2), wobei das Nanopartikelnetzwerk durch Bilden eines im Wesentlichen dreidimensionalen Netzwerks von Nanopartikeln (3) gebildet wird, die in das Matrixpolymer (2) einverleibt werden und miteinander und/oder mit dem Matrixpolymer (2) wechselwirken, wobei der Reiz Wechselwirkungen der Nanopartikel (3) miteinander und/oder zwischen den Nanopartikeln (3) und dem Matrixpolymer (2) beeinflusst,

wobei die Nanopartikel (3) Nanofasern umfassen.

**16.** Verfahren zum Induzieren einer Steifigkeitsveränderung in einer medizinischen Injektionsvorrichtung (4) mit umschaltbaren mechanischen Eigenschaften und umfassend einen hohlen Rohrabschnitt (40) mit einer Innenbohrung (400), wobei der hohle Rohrabschnitt (40) wenigstens teilweise aus einem Material besteht, das umschaltbare mechanische Eigenschaften aufweist, so dass das Material in einem ersten Schaltzustand eine erste Steifigkeit aufweist, die durch einen ersten Zug-Speichermodul (E') gekennzeichnet ist, in einem zweiten Schaltzustand eine zweite Steifigkeit aufweist, die durch einen zweiten Zug-Speichermodul (E') gekennzeichnet ist, der kleiner als der erste Zug-Speichermodul ist und von dem ersten Schaltzustand in den zweiten Schaltzustand umschaltbar ist, indem das Material gegenüber einem Reiz exponiert wird,
**dadurch gekennzeichnet,**
**dass** das Material ein Polymer-Nanokomposit (1) umfasst, umfassend

- ein Matrixpolymer (2) und
- ein Nanopartikelnetzwerk, wobei das Nanopartikelnetzwerk durch Bilden eines im Wesentlichen dreidimensionalen Netzwerks von Nanopartikeln (3) gebildet ist, die in das Matrixpolymer (2) einverleibt sind und miteinander und/oder mit dem Matrixpolymer (2) wechselwirken,
wobei der Reiz das Polymer-Nanokomposit zwischen dem ersten Schaltzustand und dem zweiten Schaltzustand umschaltet, indem er Wechselwirkungen der Nanopartikel (3) miteinander und/oder zwischen den Nanopartikeln (3) und dem Matrixpolymer (2) beeinflusst,

wobei die Nanopartikel (3) Nanofasern umfassen.

## Revendications

**1.** Dispositif (4) d'injection médical destiné à guider un liquide possédant des propriétés mécaniques pouvant changer, qui comporte une section (40) de tube creuse présentant un alésage (400) interne, dans lequel la section (40) de tube creuse est constituée au moins partiellement d'un matériau présentant des propriétés mécaniques pouvant changer de sorte que le matériau dans un premier état de changement comporte une première rigidité **caractérisée par** un premier module (E') de conservation à la traction, dans un second état de changement il comporte une seconde rigidité **caractérisée par** un second module (E') de conservation à la traction inférieur au premier module de conservation à la traction et peut changer du premier état de changement au second état de changement en exposant le matériau à un stimulus,
**caractérisé en ce que**
ledit matériau comporte un nanocomposite (1) polymère comportant

- un polymère (2) matriciel et
- un réseau de nanoparticules, dans lequel le réseau de nanoparticules est formé par une formation d'un réseau sensiblement tridimensionnel de nanoparticules (3) qui sont incorporées dans le polymère (2) matriciel et agissent réciproquement les unes avec les autres et/ou avec le polymère (2) matriciel,

dans lequel lesdites nanoparticules (3) comportent des nanofibres, et
dans lequel ledit stimulus influence lesdites interactions des nanoparticules (3) entre elles et/ou entre les nanoparticules (3) et le polymère (2) matriciel.

2. Dispositif (4) d'injection médical selon la revendication 1, dans lequel le premier module (E') de conservation à la traction est supérieur à 3,5 GPa, de préférence 6 GPa, de manière encore plus souhaitable supérieur à 9 GPa, et/ou dans lequel le second module (E') de conservation à la traction est inférieur à 1 GPa.

3. Dispositif (4) d'injection médical selon l'une quelconque des revendications 1 à 2, dans lequel le nanocomposite (1) polymère peut être obtenu en appliquant un procédé comportant les étapes consistant à :

   - fournir le polymère (2) matriciel,
   - incorporer le réseau de nanoparticules dans le polymère (2) matriciel afin d'obtenir un mélange intermédiaire,
   - soumettre le mélange intermédiaire à une étape de traitement à la chaleur mettant en jeu une exposition à une température égale ou supérieure à 100 °C.

4. Dispositif (4) d'injection médical selon la revendication 3, dans lequel l'étape de traitement à la chaleur comprend le moulage par compression du nanocomposite (1) polymère à une température égale ou supérieure à 100 °C.

5. Dispositif (4) d'injection médical selon la revendication 4 ou 5,
   dans lequel, avant l'étape de traitement à la chaleur, le mélange intermédiaire est soumis à une étape de séchage mettant en jeu une exposition à une température élevée de 30 à 80 °C sur une période de temps rallongée de 1 à 10 jours et/ou
   dans lequel l'étape de séchage comprend

   - une première étape de séchage comprenant l'exposition à une première température de séchage de 30 à 50 °C, par exemple 35 °C, sur une première période de temps allant de 1 à 10 jours, par exemple 5 jours, et une
   - seconde étape de séchage comprenant l'exposition à une seconde température de séchage de 50 à 80 °C, par exemple 70 °C, sur une seconde période de temps de 5 à 48 heures, par exemple 24 heures.

6. Dispositif (4) d'injection médical selon l'une quelconque des revendications précédentes, dans lequel le polymère (2) matriciel comporte un polymère capable de former des interactions non-covalentes, en particulier des liaisons hydrogène, avec les nanoparticules (3).

7. Dispositif (4) d'injection médical selon l'une quelconque des revendications précédentes, dans lequel ledit polymère (2) matriciel comporte au moins un polymère de vinyle tel qu'un alcool polyvinylique, un poly(acide acrylique), un poly(acrylamide), une pyridine de polyvinyle, un copolymère d'au moins un de ces monomères, un polycondensat tel qu'un polyamide ou un polyester, un polysaccharide tel que de la cellulose, de l'amidon, des alginates, des pectines, du hyaluronane, de la chitine, du chitosane ou au moins un de ses dérivés, et/ou une protéine.

8. Dispositif (4) d'injection médical selon l'une quelconque des revendications précédentes, dans lequel les nanoparticules (3) sont capables de former des interactions non-covalentes, en particulier des liaisons hydrogène, les unes avec les autres et/ou avec le polymère (2) matriciel.

9. Dispositif (4) d'injection médical selon la revendication 9, dans lequel les nanofibres (3) comportent des nanocristallites de cellulose, de préférence dans lequel les nanofibres (3) comportent des nanocristallites de cellulose dérivées de tuniciers ou de coton.

10. Dispositif (4) d'injection médical selon l'une quelconque des revendications 9 à 10, dans lequel les nanofibres (3) comportent un rapport longueur/largeur supérieur à 5, de préférence supérieur à 10, de manière encore plus souhaitable supérieur à 50, idéalement supérieur à 80.

11. Dispositif (4) d'injection médical selon l'une quelconque des revendications précédentes, dans lequel le nanocomposite (1) polymère comporte une teneur de 1 à 30 %, de préférence 2 à 25 %, de préférence encore 3 à 20 % v/v de nanoparticules (3).

**12.** Dispositif (4) d'injection médical selon l'une quelconque des revendications précédentes, dans lequel le nanocomposite (1) polymère peut changer du premier état de changement au deuxième état de changement par un stimulus qui réduit les interactions des nanoparticules (3) entre elles et/ou entre les nanoparticules (3) et le polymère (2) matriciel, et le nanocomposite (1) polymère peut changer du second état de changement au premier état de changement par un stimulus qui favorise les interactions des nanoparticules (3) entre elles et/ou entre les nanoparticules (3) et le polymère (2) matriciel.

**13.** Dispositif (4) d'injection médical selon l'une quelconque des revendications précédentes,
dans lequel le nanocomposite (1) polymère peut être changé du premier état de changement au second état de changement en soumettant le nanocomposite (1) polymère à une composition contenant de l'eau et/ou
dans lequel le nanocomposite (1) polymère peut être changé en soumettant le nanocomposite (1) polymère à une composition à une température élevée entre 30 et 50 °C, de préférence de 35 à 40 °C.

**14.** Dispositif (4) d'injection médical selon l'une quelconque des revendications précédentes,
dans lequel le rapport des modules de conservation des deux états de changement est supérieur à 20, de préférence supérieur à 50, de préférence encore supérieur à 100, et idéalement supérieur à 500 et/ou
dans lequel le nanocomposite (3) polymère présente des propriétés mécaniques essentiellement isotropes et/ou
dans lequel la section (40) de tube creuse a une épaisseur de paroi se situant entre 50 et 1000 $\mu$m, de préférence entre 100 et 500 $\mu$m.

**15.** Procédé destiné à produire un dispositif (4) d'injection médical ayant des propriétés mécaniques pouvant changer, comportant une section (40) de tube creuse présentant un alésage (400) interne, dans lequel la section (40) de tube creuse est constituée au moins partiellement d'un matériau présentant des propriétés mécaniques pouvant changer de sorte que le matériau dans un premier état de changement comporte une première rigidité **caractérisée par** un premier module (E') de conservation à la traction, dans un second état de changement il comporte une seconde rigidité **caractérisée par** un second module (E') de conservation à la traction inférieur au premier module de conservation à la traction et pouvant changer d'un premier état de changement au second état de changement en exposant le matériau à un stimulus,
**caractérisé en ce que**
ledit matériau comporte un nanocomposite polymère qui est produit en

- fournissant un polymère (2) matriciel et
- incorporant un réseau de nanoparticules dans le polymère (2) matriciel, dans lequel le réseau de nanoparticules est formé par une formation d'un réseau sensiblement tridimensionnel de nanoparticules (3) qui sont incorporées dans le polymère (2) matriciel et agissent réciproquement les unes avec les autres et/ou avec le polymère (2) matriciel,
dans lequel ledit stimulus influence les interactions des nanoparticules (3) entre elles et/ou entre les nanoparticules (3) et le polymère (2) matriciel.

dans lequel lesdites nanoparticules (3) comportent des nanofibres.

**16.** Procédé destiné à induire un changement de rigidité dans un dispositif (4) d'injection médical ayant des propriétés mécaniques pouvant changer et comportant une section (40) de tube creuse présentant un alésage (400) interne, dans lequel la section (40) de tube creuse est constituée au moins partiellement d'un matériau présentant des propriétés mécaniques pouvant changer de telle sorte que le matériau dans un premier état de changement comporte une première rigidité **caractérisée par** un premier module (E') de conservation à la traction, dans un second état de changement il comporte une seconde rigidité **caractérisée par** un second module (E') de conservation à la traction inférieur au premier module de conservation à la traction et est changé du premier état de changement au second état de changement en exposant le matériau à un stimulus, **caractérisé en ce que**
ledit matériau comporte un nanocomposite polymère comportant

- un polymère (2) matriciel et
- un réseau de nanoparticules, dans lequel le réseau de nanoparticules est formé par une formation d'un réseau sensiblement tridimensionnel de nanoparticules (3) qui sont incorporées dans le polymère (2) matriciel et agissent réciproquement les unes avec les autres et/ou avec le polymère (2) matriciel,
- dans lequel ledit stimulus fait changer le nanocomposite polymère entre le premier état de changement et le second état de changement en influençant les interactions des nanoparticules (3) entre elles et/ou entre les nanoparticules (3) et le polymère (2) matriciel,

dans lequel lesdites nanoparticule (3) comportent des nanofibres.

FIG 1A

FIG 1B

FIG 2A

FIG 2B

## FIG 2C

Legend:
- △ — Neat PVOH
- ■ — 4% v/v PVOH/CNW
- ○ — 8% v/v PVOH/CNW
- ▲ — 12% v/v PVOH/CNW
- □ — 16% v/v PVOH/CNW

Y-axis: E' (Pa), X-axis: Temperature (°C)

## FIG 2D

Legend:
- ● — Neat PVOH
- ■ — 4% v/v PVOH/CNW
- □ — 8% v/v PVOH/CNW
- ▲ — 12% v/v PVOH/CNW
- △ — 16% v/v PVOH/CNW

Y-axis: tan δ, X-axis: Temperature (°C)

FIG 3A

FIG 3B

FIG 4

FIG 5A

FIG 5B

FIG 6A

FIG 6B

FIG 7A

FIG 7B

$y = -58.1x + 803.6$
$R^2 = 0.99$

$y = 23.3x + 5.2$
$R^2 = 0.99$

FIG 7C

$y = -81.7x + 845$
$R^2 = 0.99$

$y = 15.7x + 128$
$R^2 = 0.99$

FIG 8A                                    FIG 8B

FIG 8C                                    FIG 8D

FIG 9A

FIG 9B

FIG 10

FIG 11

FIG 12

**FIG 13**

L

E

41

42

4

α

40

400

**FIG 14**

4

5

41

FIG 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4755173 A
- US 7435240 B
- US 7513891 B
- WO 2006074948 A1
- US 20090318590 A1

### Non-patent literature cited in the description

- **CAPADONA, J. R. et al.** *Science,* 2008, vol. 319, 1370
- **SHANMUGANATHAN K. et al.** Bio-inspired mechanically-adaptive nanocomposites derived from cotton cellulose whiskers. *J. Mater. Chem.,* 2010, vol. 20, 180-186
- **KIM, H. ; COLTON, J.S.** *Journal of Medical Engineering and Technology,* 2005, vol. 29, 181
- **SIQUEIRA, G. et al.** *Polymers,* 2010, vol. 2, 728
- **KLEMM, D. et al.** *Angew. Chem. Int. Ed.,* 2011, vol. 50, 5438
- **FAVIER et al.** *Macromolecules,* 1995, vol. 28, 6365
- **SHANMUGANATHAN et al.** *ACS Appl. Mater. Interfaces,* 2010, vol. 2, 165
- **CAPADONA, J. R. et al.** *Biomacromolecules,* 2009, vol. 10, 712
- **SAMIR, M. A. et al.** *Biomacromolecules,* 2005, vol. 6, 612
- **SHANMUGANATHAN, K. et al.** *J. Mater. Chem.,* 2010, vol. 20, 180
- **HAJJI, P. et al.** *Polym. Compos.,* 1996, vol. 17, 612